# EUROPEAN PATENT APPLICATION

(11) **EP 4 767 996 A2**
(43) Date of publication of application: **01.07.2026**
(21) Application number: 26178757.6
(22) Date of filing: 23.11.2021
(51) Int. Cl.: A61F 2/24

(54) **SYSTEMS AND APPARATUSES FOR REMOVING A MEDICAL IMPLANT FROM CARDIAC TISSUE**

(30) Priority: 30.11.2020 US 202063119317 P; 22.11.2021 US 202117532559
(62) Divisional of application: 21827758.0
(71) Applicant: Evalve, Inc., Santa Clara, CA 95054 (US)
(72) Inventor: The designation of the inventor has not yet been filed
(74) Representative: Boult Wade Tennant LLP

(57) **Abstract**

An implant removal device having an elongate body having a proximal end and a distal end, the elongate body being resiliently flexible and configured to transmit torque from the proximal end to the distal end with a predetermined turning ratio, and a capturing structure extending distally from the distal end and having a capture region, the capturing structure being configured to selectively center a deployed implant in relation to a longitudinal axis of the elongate body and the capture region to aid with capture and subsequent removal.

## Description

### CROSS-REFERENCE TO RELATED APPLICATIONS

This application claims the benefit of and priority to U.S. Non-Provisional Patent Application No. 17/532,559, filed November 22, 2021, entitled "SYSTEMS, APPARATUSES, AND METHODS FOR REMOVING A MEDICAL IMPLANT FROM CARDIAC TISSUE," which claims the benefit of and priority to U.S. Provisional Patent Application No. 63/119,317, filed November 30, 2020, entitled "SYSTEMS, APPARATUSES, AND METHODS FOR REMOVING A MEDICAL IMPLANT FROM CARDIAC TISSUE," the entire contents of which are incorporated by reference herein.

### BACKGROUND OF THE INVENTION

### 1. The Field of the Invention

The present disclosure relates generally systems, apparatuses, and methods for removal of a medical implant from cardiac tissue, such as a medical implant attached to a valve leaflet.

### 2. The Relevant Technology

The present invention relates generally to medical methods, devices, and systems. In particular, the present invention relates to methods, devices, and systems for the endovascular, percutaneous, or minimally invasive surgical treatment of bodily tissues, such as tissue approximation or valve repair. More particularly, the present invention relates to repair of valves of the heart and venous valves, and devices and methods for removing or disabling mitral valve repair components through minimally invasive procedures.

Surgical repair of bodily tissues often involves tissue approximation and fastening of such tissues in the approximated arrangement. When repairing valves, tissue approximation includes coapting the leaflets of the valves in a therapeutic arrangement which may then be maintained by fastening or fixing the leaflets. Such coaptation can be used to treat regurgitation, which most commonly occurs in the mitral valve.

Mitral valve regurgitation is characterized by retrograde flow from the left ventricle of a heart through an incompetent mitral valve into the left atrium. During a normal cycle of heart contraction (systole), the mitral valve acts as a check valve to prevent the flow of oxygenated blood back into the left atrium. In this way, the oxygenated blood is pumped into the aorta through the aortic valve. Regurgitation of the valve can significantly decrease the pumping efficiency of the heart, placing the patient at risk of severe, progressive heart failure.

Mitral valve regurgitation can result from a number of different mechanical defects in the mitral valve or the left ventricular wall. The valve leaflets, the valve chordae which connect the leaflets to the papillary muscles, the papillary muscles themselves, or the left ventricular wall may be damaged or otherwise dysfunctional. Commonly, the valve annulus may be damaged, dilated, or weakened, limiting the ability of the mitral valve to close adequately against the high pressures of the left ventricle.

The most common treatments for mitral valve regurgitation rely on valve replacement or repair, including leaflet and annulus remodeling, the latter generally referred to as valve annuloplasty. One technique for mitral valve repair, which relies on suturing adjacent segments of the opposed valve leaflets together, is referred to as the "bow-tie" or "edge-to-edge" technique. While all these techniques can be effective, they usually rely on open-heart surgery where the patient's chest is opened, typically via a sternotomy, and the patient placed on cardiopulmonary bypass. The need to both open the chest and place the patient on bypass is traumatic and has associated high mortality and morbidity.

In some patients, a fixation device can be installed into the heart using minimally invasive techniques. The fixation device can hold the adjacent segments of the opposed valve leaflets together and may reduce mitral valve regurgitation. One such device used to clip the anterior and posterior leaflets of the mitral valve together is the MitraClip^{®} fixation device, sold by Abbott Vascular, Santa Clara, Calif., USA.

However, sometimes after a fixation device is installed, undesirable mitral valve regurgitation can still exist, or can arise again. Further, other problems with the heart may arise that can make it desirable for the fixation device to be disabled or removed, usually in order that other procedures may be performed on the heart.

Current techniques for removing or disabling mitral valve fixation devices usually rely on open-heart surgery where the patient's chest is opened, typically via a sternotomy, and the patient placed on cardiopulmonary bypass.

For these reasons, it would be desirable to provide alternative and additional methods, devices, and systems for removing or disabling fixation devices that are already installed. Such methods, devices, and systems should preferably not require open chest access and be capable of being performed either endovascularly, i.e., using devices that are advanced to the heart from a point in the patient's vasculature remote from the heart or by another minimally invasive approach. The methods, devices, and systems may be useful for the repair of tissues in the body other than heart valves. At least some of these objectives will be met by the inventions described hereinbelow.

### BRIEF SUMMARY OF THE INVENTION

These and other objects and features of the present invention will become more fully apparent from the following description and appended claims or may be learned by the practice of the invention as set forth hereinafter.

The present disclosure describes methods and devices that may be employed after a device that clips the anterior and posterior leaflets of the mitral valve together has been installed.

Sometimes after such a device is installed in the heart, problems may still exist or could arise with the function of the mitral valve or with the heart generally. In order to resolve these problems, it may be desirable to remove or disable the previously implanted device. It may also be desirable to perform a procedure on the mitral valve, such as mitral valve annuloplasty, balloon valvuloplasty, mitral valve repair, or installation of a replacement valve. In order to be able to perform procedures on a heart that already has a mitral valve fixation device attached thereto, it may be desirable to first remove or disable the device.

Traditionally, mitral valve fixation devices have been removed through invasive surgeries, such as open-heart surgery. However, less invasive methods would be preferable, because, for example, persons with a mitral valve fixation device may not be suitable candidates for invasive surgery. Disclosed herein are methods and devices that may be used in disabling or removing such a device.

For example, according to an embodiment, a method of removing a fixation device that holds anterior and posterior leaflets of the mitral valve together is disclosed. The method may include surrounding a portion of the fixation device with a capturing structure, enclosing the captured fixation device, separating the fixation device from the cardiac tissue, and removing the fixation device from the patient.

According to another embodiment, a method of removing a fixation device may include cutting one leaflet along or near the engagement of the fixation device with the leaflet so that the fixation device separates from a main portion of that leaflet from which it is cut.

Another method for removing a fixation device may include accessing, through an endovascular procedure, the fixation device holding the anterior and posterior leaflets of the mitral valve together. The endovascular procedure may advance a capturing structure, such as a portion of a braided structure or a finger conical structure, through the vasculature of the patient, and into the heart. Following capturing a portion of the fixation device with the capturing structure, the fixation device may be separated (e.g., cut) from both leaflets with a removal tool that at least partially surrounds the capturing structure and the fixation device. The fixation device may then be removed from the body of the patient.

Any of such described methods may advantageously be performed with minimal invasion, e.g., through an endovascular procedure that advances any devices employed in the procedure (e.g., tools for capturing the fixation device, cutting or otherwise separating the fixation device and/or surrounding tissue) through the vasculature of the patient, into the heart, where the devices may access the mitral valve.

Another embodiment according to the present disclosure is directed to a system for removing a mitral valve fixation device. The system may include an implant management tool with cutting means disposed at the distal end, the cutting means being configured to cut the tissue surrounding the installed fixation device. The system may further include an implant removal device with a capturing structure, such as a retaining means, disposed at the distal end. The capturing structure may be configured to retain the fixation device and/or cut portions thereof, so as to allow its removal using the implant management tool.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:
FIG. 1 illustrates the left ventricle and left atrium of the heart during systole.
FIG. 2A illustrates free edges of leaflets of the mitral valve in normal coaptation, and FIG. 2B illustrates the free edges in regurgitative coaptation.
FIG. 3 illustrates the position of the fixation device in a desired orientation relative to the leaflets.
FIG. 4 is another illustration of the position of the fixation device in a desired orientation relative to the leaflets.
FIG. 5 is an illustration of the position of the fixation device in a desired orientation relative to the leaflets following tissue ingrowth.
FIG. 6A illustrates a system for removing a fixation device that may include a braided capture structure according to configurations of the invention.
FIG. 6B illustrates a system for removing a fixation device that may include a finger conical capture structure according to configurations of the invention.
FIG. 6C a system for removing a fixation device that may include a braided capture structure and a finger conical capture structure according to configurations of the invention
FIG. 6D illustrates a portion of the system of FIG. 6A,FIG. 6B, or FIG. 6C with a proximal end of a cutting member mounted to a cutting member actuator according to a configuration of the invention.
FIGS. 7A-7F illustrate various distal configurations of a cutting member according to various configurations of the invention.
FIG. 8A is a cross-sectional view of a braided capturing structure of the system of FIG. 6A in an extended state according to one configuration of the invention.
FIG. 8B is a cross-sectional view of a braided capturing structure of the system of FIG. 6A in a partially retracted state according to one configuration of the invention.
FIG. 9 is an enlarged view of a braided capturing structure of FIGS. 8A and 8B according to one configuration of the invention.
FIGS. 10A-10F illustrate alternate cross-sections for a thread of a braided capturing structure of FIG. 9 or a finger of the finger conical capturing structure of FIGS. 12A and 12 B according to configurations of the invention.
FIG. 11A is a cross-sectional view of an implant removal device having a braided capturing structure of FIGS. 8A and 8B according to one configuration of the invention.
FIG. 11B is a partial cross-section view of an implant removal device having a braided capturing structure of FIGS. 8A and 8B, including an optional sheath according to one configuration of the invention.
FIG. 12A is a cross-sectional view of a finger conical capturing structure of the system of FIG. 6B in an extended state according to one configuration of the invention.
FIG. 12B is a cross-sectional view of a finger conical capturing structure of the system of FIG. 6B in a partially retracted state according to one configuration of the invention.
FIG. 13A is a cross-sectional view of an implant removal device having a finger conical capturing structure of FIGS. 12A and 12B according to one configuration of the invention.
FIG. 13B is a cross-sectional view of an implant removal device having a finger conical capturing structure of FIGS. 12A and 12B, including an optional sheath according to one configuration of the invention.
FIG. 14A illustrates an alternate configuration of an implant removal device having a hypotube sheath according to one configuration of the invention.
FIG. 14B illustrates an alternate configuration of an implant removal device having a braided sheath according to one configuration of the invention.
FIG. 15 is a cut-away view of a heart with a portion of an implant management tool according to the present invention.
FIG. 16 a cut-away view of a heart with a portion of an implant management tool according to the present invention.
FIG. 17 a cut-away view of a heart with a portion of an implant management tool according to the present invention
FIG. 18 illustrates an implant management tool advanced toward a fixation device that was previously implanted on leaflets.
FIG. 19 illustrates an implant removal tool advanced from the implant management tool towards the fixation device of FIG. 17.
FIG. 20 illustrates a capturing structure of an implant removal tool identifying and locating the fixation device of FIG. 17.
FIG. 21 illustrates a capturing structure of an implant removal tool capturing the fixation device of FIG. 17.
FIG. 22 illustrates an implant removal tool, with captured fixation device, withdrawn into the implant management tool of FIG. 17 and cutting the captured fixation device from the leaflets.
FIG. 23 illustrates an alternate configuration of an implant removal device having an RF cutting element according to one configuration of the invention.
FIG. 24 illustrates an alternate configuration or a braided capturing structure having a cutting surface at a distal end thereof according to one configuration of the invention.
FIG. 25 illustrates an alternate configuration of a finger conical capturing structure having a cutting surface at a distal end thereof according to one configuration of the invention.
FIG. 26 illustrates an alternate configuration of a combination of a braided capturing structure and a finger conical capturing structure having one or more cutting surface(s) at a distal end thereof according to one configuration of the invention.

### DETAILED DESCRIPTION OF THE INVENTION

One or more specific embodiments of the present disclosure will be described below. In an effort to provide a concise description of these embodiments, some features of an actual embodiment may be described in the specification. It should be appreciated that in the development of any such actual embodiment, as in any engineering or design project, numerous embodiment-specific decisions will be made to achieve the developers' specific goals, such as compliance with system-related and business-related constraints, which may vary from one embodiment to another. It should further be appreciated that such a development effort might be complex and time consuming, but would nevertheless be a routine undertaking of design, fabrication, and manufacture for those of ordinary skill having the benefit of this disclosure.

One or more embodiments of the present disclosure may generally relate to apparatuses, systems, and methods to remove a fixation device deployed to a target location. The apparatuses, systems, and methods can be used to separate a fixation device coapting leaflet tissue, such as the mitral valve leaflets, and then remove the fixation device. Following fixation device removal, a replacement valve or other device can subsequently be deployed to replace the mitral valve, for instance. Illustrative fixation devices can include, but not limited to, MitraClip^{®}.

While the present disclosure will describe a particular implementation of apparatuses and systems, with associated methods, for removing the fixation device, it should be understood that any of systems, apparatuses, and methods described herein may be applicable to other uses, including and not limited to removing fixation devices positioned in other locations with a patient's anatomy. Additionally, elements described in relation to any embodiment depicted and/or described herein may be combinable with elements described in relation to any other embodiment depicted and/or described herein.

### I. Introduction

### A. Cardiac Physiology

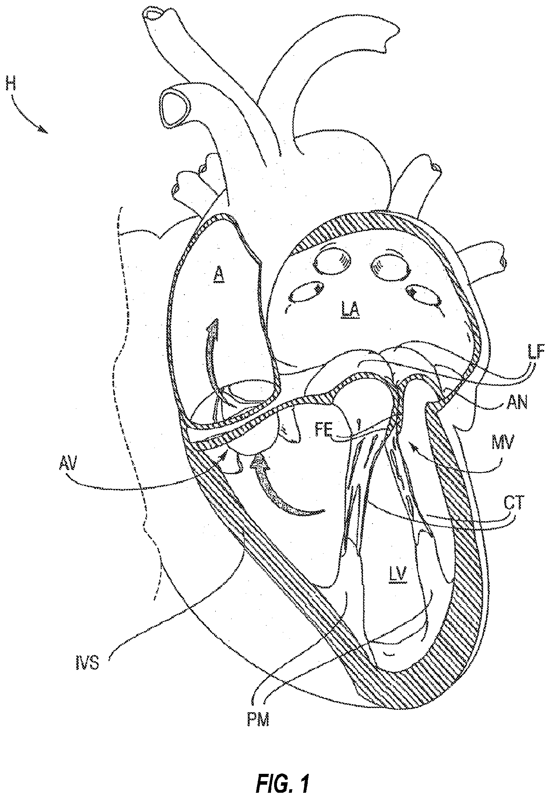

The left ventricle (LV) of a normal heart H in systole is illustrated in FIG. 1. The left ventricle (LV) is contracting and blood flows outwardly through the tricuspid (aortic) valve (AV) in the direction of the arrows. Back flow of blood or "regurgitation" through the mitral valve (MV) is prevented since the mitral valve is configured as a "check valve" which prevents back flow when pressure in the left ventricle is higher than that in the left atrium (LA). The mitral valve (MV) comprises a pair of leaflets having free edges (FE) which meet evenly to close, as illustrated in FIG. 1. The opposite ends of the leaflets (LF) are attached to the surrounding heart structure along an annular region referred to as the annulus (AN). The free edges (FE) of the leaflets (LF) are secured to the lower portions of the left ventricle LV through chordae tendineae (CT) (referred to hereinafter as the chordae) which include a plurality of branching tendons secured over the lower surfaces of each of the valve leaflets (LF). The chordae tendineae (CT) in turn, are attached to the papillary muscles (PM) which extend upwardly from the lower portions of the left ventricle and intraventricular septum IVS.

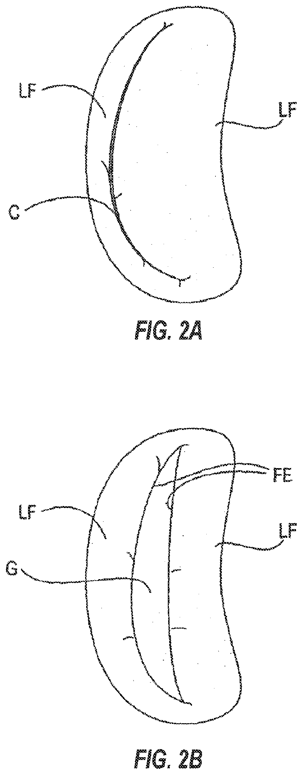

A number of structural defects in the heart can cause mitral valve regurgitation. Regurgitation occurs when the valve leaflets do not close properly allowing leakage from the ventricle into the atrium. As shown in FIG. 2A, the free edges of the anterior and posterior leaflets normally meet along a line of coaptation (C). An example of a defect causing regurgitation is shown in FIG. 2B. Here an enlargement of the heart causes the mitral annulus to become enlarged, making it impossible for the free edges (FE) to meet during systole. This results in a gap (G) which allows blood to leak through the valve during ventricular systole. Ruptured or elongated chordae can also cause a valve leaflet to prolapse since inadequate tension is transmitted to the leaflet via the chordae. While the other leaflet maintains a normal profile, the two valve leaflets do not properly meet and leakage from the left ventricle into the left atrium will occur. Such regurgitation can also occur in patients who have suffered ischemic heart disease where the left ventricle does not contract sufficiently to effect proper closure.

### II. General Overview of Fixation Technology

Fixation devices are used for grasping, approximating and fixating tissues such as valve leaflets to treat cardiac valve regurgitation, particularly mitral valve regurgitation. In some cases, the fixation devices may also provide features that allow repositioning and removal of the device if so desired, particularly in areas where removal may be hindered by anatomical features such as chordae CT. Such removal would allow the surgeon to reapproach the valve in a new manner if so desired.

Grasping will preferably be atraumatic providing a number of benefits. By atraumatic, it is meant that the devices and methods may be applied to the valve leaflets and then removed without causing any significant clinical impairment of leaflet structure or function. The leaflets and valve continue to function substantially the same as before the fixation devices are applied. Thus, some minor penetration or denting of the leaflets may occur using the devices while still meeting the definition of "atraumatic." Similarly, during disabling or removal of the fixation device, a small portion of the leaflet(s) may be cut around the edges of the fixation device. Such atraumatic installation, disabling, or removal enables the devices to be applied to a diseased valve and, if desired, removed or repositioned without having negatively affected valve function. In addition, it will be understood that in some cases it may be necessary or desirable to pierce or otherwise permanently affect the leaflets during either grasping, fixing and/or removal. In some cases, grasping and fixation may be accomplished by a single device.

The fixation devices may rely upon the use of an interventional tool that is positioned near a desired treatment site and used to grasp the target tissue. In endovascular applications, the interventional tool is typically an interventional catheter. In surgical applications, the interventional tool is typically an interventional instrument. Fixation of the grasped tissue is accomplished by maintaining grasping with a portion of the interventional tool which is left behind as an implant. The fixation devices are well adapted for the repair of valves, especially cardiac valves such as the mitral valve.

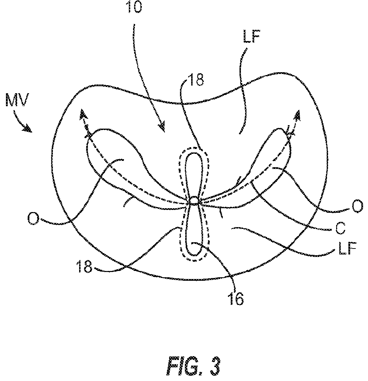

FIG. 3 illustrates the position of a fixation device 10 in a desired orientation in relation to the leaflets LF. Additional description regarding such fixation may be found in U.S. Patent No. 9,572,666, the disclosure of which is incorporated herein by reference in its entirety. The illustrated view is a short-axis view of the mitral valve MV from the atrial side, therefore, the proximal elements 12 are shown in solid line and the distal elements 14 are shown in dashed line. When describing the devices of the invention herein, "proximal" shall mean the direction toward the end of the device to be manipulated by the user outside the patient's body, and "distal" shall mean the direction toward the working end of the device that is positioned at the treatment site and away from the user. With respect to the mitral valve, proximal shall refer to the atrial or upstream side of the valve leaflets and distal shall refer to the ventricular or downstream side of the valve leaflets.

The proximal and distal elements 12, 14 are positioned to be substantially perpendicular to the line of coaptation C. The device 10 may be moved roughly along the line of coaptation to the location of regurgitation. The leaflets LF are held in place so that during diastole, as shown in FIG. 3, the leaflets LF remain in position between the elements 12, 14 surrounded by openings or orifices O which result from the diastolic pressure gradient. Advantageously, leaflets LF are coapted such that their proximal or upstream surfaces are facing each other in a vertical orientation, parallel to the direction of blood flow through mitral valve MV. The upstream surfaces may be brought together so as to be in contact with one another or may be held slightly apart, but will preferably be maintained in the vertical orientation in which the upstream surfaces face each other at the point of coaptation. This simulates the double orifice geometry of a standard surgical bow-tie repair. Color Doppler echo will show if the regurgitation of the valve has been reduced. If the resulting mitral flow pattern is satisfactory, the leaflets may be fixed together in this orientation. If the resulting color Doppler image shows insufficient improvement in mitral regurgitation, the fixation device 10 may be repositioned. This may be repeated until an optimal result is produced wherein the leaflets LF are held in place.

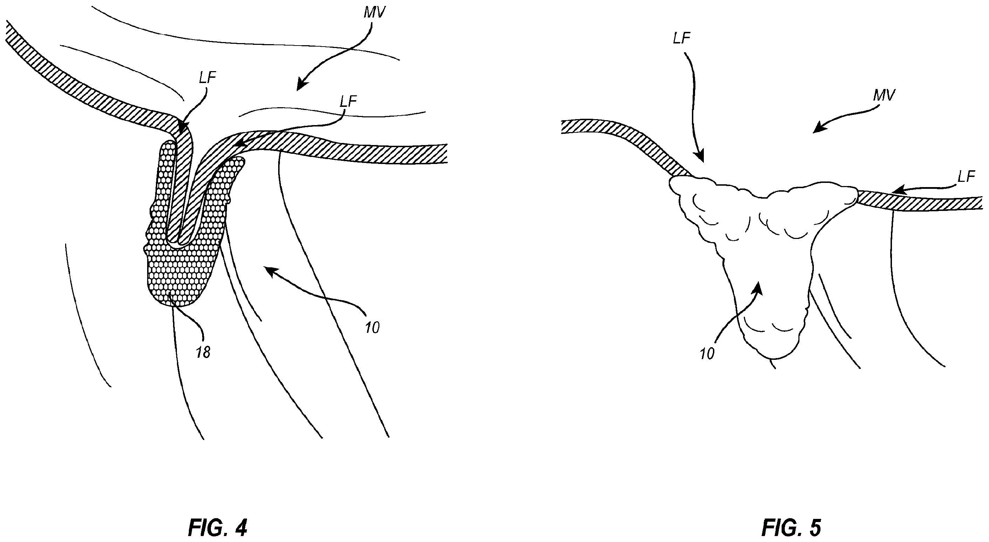

FIGS. 4 and 5 illustrate the fixation device 10 once deployed. As illustrated, the fixation device 10 may optionally include a covering, such as covering 16. The covering 16 may assist in grasping the tissue and may later provide a surface for tissue ingrowth, such as illustrated in FIG. 5. Ingrowth of the surrounding tissues, such as the valve leaflets, provides stability to the device 10 as it is further anchored in place and may cover the device with native tissue, thus reducing the possibility of immunologic reactions. The covering 16 may be comprised of any biocompatible material, such as polyethylene terephthalate, polyester, cotton, polyurethane, expanded polytetrafluoroethylene (ePTFE), silicone, or various polymers or fibers and have any suitable form, such as a fabric, mesh, textured weave, felt, looped or porous structure. Generally, the covering has a low profile so as not to interfere with delivery through an introducer sheath or with grasping and coapting of leaflets or tissue. Additional description regarding such coverings may be found in PCT Publication No. WO 2004/103162, the disclosure of which is incorporated herein by reference in its entirety.

### III. Methods of Removing a Fixation Device

Sometimes, after installation of a fixation device in the heart, it needs to be removed. Ordinarily, this has been done during an invasive procedure such as open-heart surgery. Invasive procedures such as these often have high risk of complications, however. Further, sometimes mitral valve fixation devices are installed on patients for whom open heart or more invasive procedures are otherwise unnecessary or undesirable. For these patients, and even for patients in whom open-heart surgery is used, it would be beneficial to have devices and systems specifically designed for removing the fixation devices within an endovascular procedure, rather than a procedure requiring open heart access.

Minimally invasive systems, methods, and devices for removing the fixation devices are disclosed herein. These minimally invasive systems, methods, and devices allow a practitioner to remove the fixation device and, optionally, then proceed to do other things in the heart, without necessarily requiring open heart access or other more invasive procedures. Such systems, methods, and devices are configured to be effective even if the fixation device has been installed for weeks, months, or years, such that tissue surrounding the device may have grown over, into, or around the fixation device. As a result of such tissue ingrowth, or for other reasons, removal of the fixation device as described above that may be practical during the initial placement procedure may no longer be practical. The systems, methods, and devices disclosed herein may also be useful for adjusting the installation of a mitral valve fixation device after it is installed.

An embodiment of the present invention discloses systems that include various devices that may include catheters and tools that perform various functions, and also multifunctional catheters and tools that can perform any combination of functions. Such functions may include holding or retaining an installed fixation device; cutting or otherwise partitioning a leaflet or leaflets; removing a fixation device; and subsequently repairing the leaflet(s) or associated valve. Related methods for performing such functions are also disclosed.

The devices and associated methods and systems described herein may be used in combination with imaging modalities such as x-ray, fluoroscopy, echocardiography, charge coupled device cameras, complementary metal oxide semiconductor cameras, magnetic resonance imaging, and other imaging modalities. The availability of such imaging modalities during such procedures may help practitioners visualize, for example, where the fixation devices are, how they are connected to the heart, and where to direct the various catheters and/or other devices.

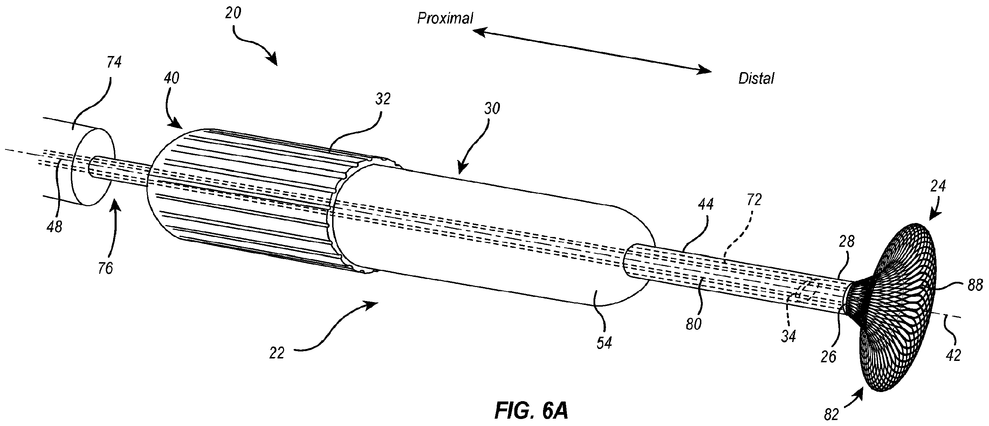

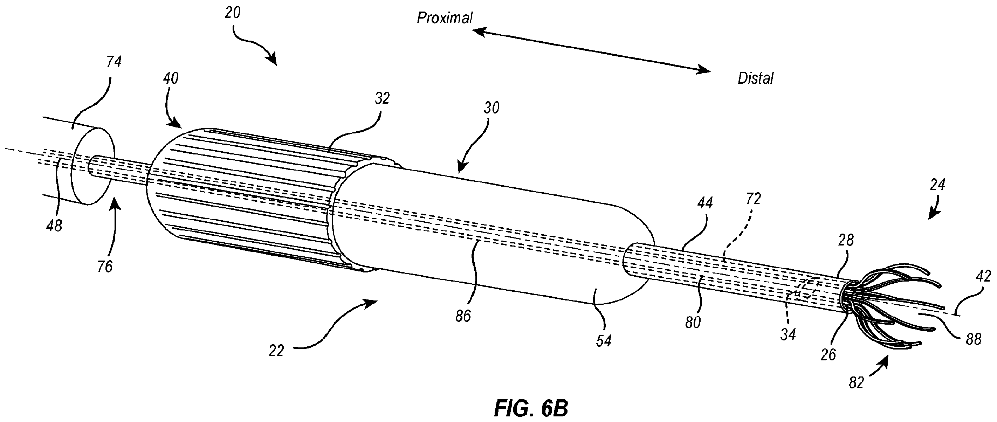

Turning to FIGS. 6A and 6B, illustrated is an implant removal system 20 that can be used to capture and subsequently remove a fixation device, such as the fixation device 10 of FIGS. 3-5. The implant removal system 20 can be used to access the fixation device within a heart cavity, such as when the fixation device 10 captures mitral valve leaflets (LF). This can be achieved through a transapical antegrade approach or transfemoral retrograde approach. While the transapical approach will be discussed in more detail hereinafter, it will be understood that the implant removal system 20 can also be used for a transfemoral approach through lengthening and increasing a flexibility of various components, providing steering capabilities, and making such other modifications that one-skilled in the art can contemplate based upon the underlying disclosure presented herein.

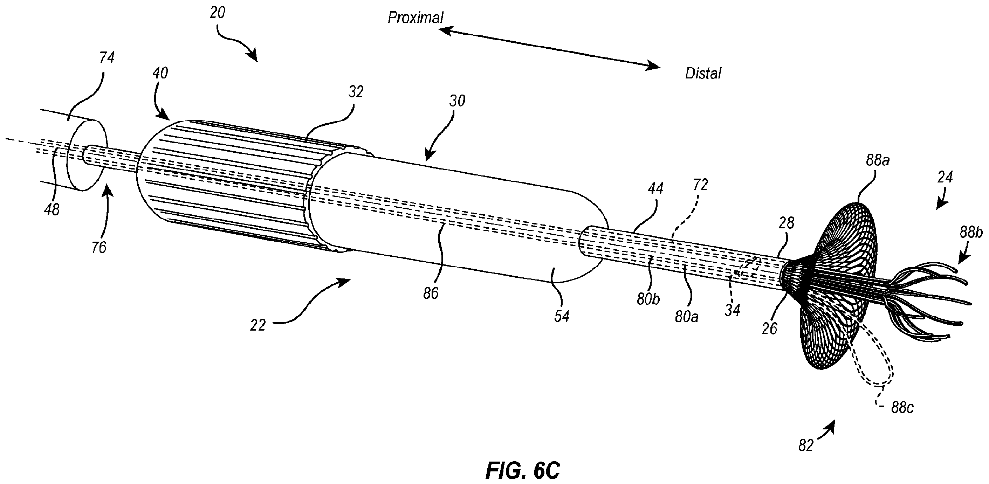

As illustrated in FIGS. 6A, 6B, and 6C the implant removal system 20 includes an implant management tool 22 and an implant capturing device 24 that can be deployed from the implant management tool 22 to position an end of the implant capturing device 24 in close proximity to a previously deployed fixation device, such as fixation device 10 (FIGS. 3-5). In the presently illustrated configuration, the implant capturing device 24 can be used to capture the fixation device 10 (FIGS. 3-5), including some ingrowth tissue, leaflet tissue or other tissue (collectively referred to herein as "tissue"), while the implant management tool 22 can be used to separate the captured fixation device 10 (FIGS. 3-5) and tissue from the surrounding anatomy. For instance, once the implant capturing device 24 captures the fixation device 10 and tissue, and the captured fixation device 10 and tissue are withdrawn through an opening 26 into a region 28 of the implant management tool 22, manipulation of an actuator 32 of a handle 30 can move a cutting member 34 to the opening 26 and cut the tissue surrounding the fixation device 10 (FIGS. 3-5). The detached fixation device 10 (FIGS. 3-5) is retained within the region 28 formed near the opening 26, which is at least partially closed by the cutting member 34. Once the fixation device 10 (FIGS. 3-5) is detached from the surrounding tissue, the implant removal system 20, with the captured fixation device 10 (FIGS. 3-5), can be removed from the patient. Thereafter, the leaflet(s) are repaired or an artificial or replacement valve is deployed, such as taught in U.S. Patent Nos. 9,895,221 and 10,6175,519 and U.S. Patent Publication No. 2018/0078370, the disclosure of which is incorporate herein by this reference.

The implant management tool 22 has a proximal end 40 and a distal end 42, with the handle 30 disposed towards the proximal end 40, with a shaft 44 extending from a handle distal end towards the distal end 42 of the implant management tool 22. The opening 26 toward the distal end 42 is coaxial with a lumen 72 of the shaft 44 or having the opening 26 opening from the distal end 42 of the shaft 44 in a direction parallel to a longitudinal axis 48 of the shaft 44.

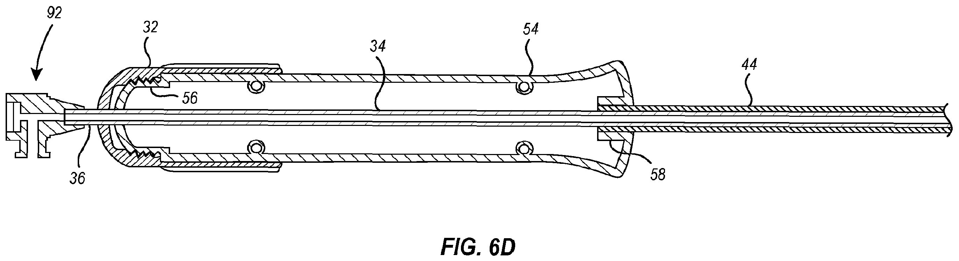

The handle 30 includes a cutting member actuator 32 rotatably mounted to a handle body 54 to rotate the cutting member 34 from within the lumen 72 in one direction or in both directions (e.g., counterclockwise or clockwise). Such rotation can include greater or lesser than 360 degrees of rotation. The cutting member 34 is a generally tubular member with a cutting edge 60 at a cutting member distal end 42. As illustrated in FIG. 6D, the handle 30 can optionally include a luer port 92 at a cutting member proximal end 36. The luer port 92 prevents air ingress through use of a hemostatic valve, such as a rotating hemostatic valve, but allowing guidewires and snares, and optionally the implant capturing device 24, to be inserted and translated without air ingress or loss of blood. In some embodiments, the handle 74 may be pushed to translate the cutting member.

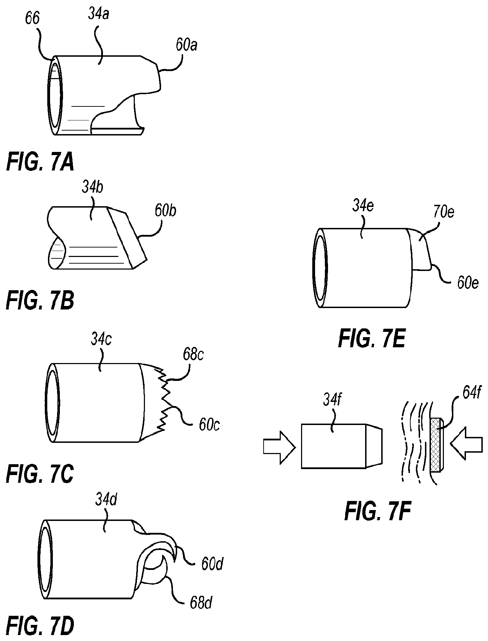

While a general discussion of the cutting member is presented above, referring now to FIGS. 7A thru 7G, exemplary embodiments of the cutting member are illustrated. For ease of explanation, the illustrated cutting members are depicted in relation to tissue without a fixation device and associated tissue. It will be understood, however, that the illustrated cutting members can be used to remove the fixation device and tissue by disposing the same with a lumen 72 of the cutting member as discussed herein.

Referring now to FIG. 7A, the cutting member 34a resembles a biopsy punch, where the cutting member 34a includes a hollow thin-walled metal cylinder 66 in which the cutting edge 60a is formed, such as a razor-like cutting edge. It should be appreciated that the cutting member 34a can have different circumferential shapes including, but not limited to, a circle, an ellipse, a rectangle, a rectangle with rounded corners, etc. The cutting edge 60a can meet the cylindrical wall perpendicularly, or at an angle, as illustrated as the cutting edge 60b of cutting member 34b (FIG. 7B).

Referring now to FIG. 7C, a cutting edge 60c of a cylindrical cutting member 34c may be serrated. The serrations 68c can be uniformly or non-uniformly distributed or arranged in a particular manner in which some serrations can optionally be of a different size and shape than others. The serrations 68c can assume the typical triangular shape, or can take other more exotic shapes like that of a sickle 68d (FIG. 7D), spade, or the like. Additionally, while each of the cutting edges can be annular so that it approximates a tubular shape, it will be understood that the sharpened portion of the cutting edge 60 and/or the end wall 64 can be formed by an arc portion 70e of a complete annular edge of the cutting edge 60e, such as illustrated in FIG. 7E.

The cutting member 34 described herein can have one or both of two types of movements: the first being a translation along the longitudinal axis of the cutting member 34 in the direction that moves the cutting edge toward tissue to be excised; and the second being rotation of the cylindrical cutting member 34 about its longitudinal axis. In the illustrated configuration of FIGS. 6A and 6B, the cutting member 34 is both translated along the longitudinal axis and rotated about its longitudinal axis, the cutting edge 60 of the cutting member 34 imparts on the target tissue both a pushing and a sliding motion, such as when cutting member actuator 32 is manipulated by a user. The cutting member 34 in this embodiment will have a circular circumferential shape to allow for the desired rotational and longitudinal movement. This movement can be achieved, in one configuration, with a proximal end of the cutting member 34 mounted to the cutting member actuator 32, such as illustrated in FIG. 6D. The handle body 54 has a threaded portion 56, and the cutting member actuator 32 has a complementary threaded portion 36, which engages with the threaded portion 56 of the handle body 54. Rotation of the cutting member actuator 32 about the threaded portion 56 of the handle body 54 allows the cutting member 34 to translate and rotate in relation to the stationary shaft 44 that is mounted to a mounting surface 58 of the handle body 54.

The cutting member 34 may be the only element of a cutting arrangement, and the one blade is advanced towards the desired tissue throughout the duration of the cutting process. For instance, the cutting member 34f is advanced toward the end wall 64f with the end wall 64f functioning like a backstop, cutting mat, or anvil for the cutting member 34f to press against, as illustrated in FIG. 7F. With the tissue resting against the end wall 64f, the end wall 64f creates a more stable cutting configuration.

It should be appreciated that a radiofrequency (RF) or an ultrasonic cutting arrangement can be used instead of the cutting member blade. The shape of the RF cutting element or the ultrasonic cutting element can adopt any of the above-described configurations.

Returning to FIGS. 6A, 6B, and 6C the implant capturing device 24 includes an elongate body 80 and a capturing structure 82. In embodiments illustrated in FIGS. 6A and 6B, the capturing structure 82 may be a braided capturing structure (see Fig. 6A) or a finger conical capturing structure (see Fig. 6B), extending from the opening 26 of a distal end of the elongate body 80. The elongate body 80 in FIGS. 6A and 6B extends from the proximal end 40, through the handle 30, the shaft 44, the lumen 72, and the cutting member 34 to exit the opening 26 at the distal end 42. With such elongation, a physician can manipulate and position the implant capturing device 24 with the patient's anatomy to capture the fixation device 10 (FIGS. 3-5) and tissue. In some embodiments, the cutting member 34 in FIGS. 6A or 6B may be disposed between the lumen 72 and the elongate body 80 inside the lumen 72.

In embodiments illustrated in FIG. 6C, the capturing structure 82 includes a combination of a braided capturing structure 88a and a finger conical capturing structure 88b. In particular, the finger capturing structure 88b is disposed within the braided conical capturing structure 88a. The elongate body 80 of FIG. 6C includes an elongate body 88a, extending from the braided capturing structure 88a, and an elongate body 88b (disposed within the elongate body 88a), extending from the finger conical capturing structure 88b. In some embodiments, each elongate body 80a or 80b can be individually controlled to extend or retract along the lumen 72. In some embodiments, the relative extension position of the elongate body 80a and the elongate body 80b may be adjusted. In yet some embodiments, the elongate bodies 80a and 80b are configured to extended or retracted simultaneously. In other embodiments, instead of the finger conical capturing structure 88b, a snare 88c (illustrated in phantom) could be used for capturing the fixation device 10. In still other embodiments, the snare 88c can be used with the finger conical capturing structure 88b without the braided conical capturing structure 88a. More generally, one or more of the braided conical capturing structure 88a, the finger conical capturing structure 88b, and snare 88c can be used to individually or collectively to capture the fixation device 10.

The cutting member 34 may be disposed between the elongate body 80a (extending from the braided capture structure 88a) and the elongate body 80b (extending from the finger conical capturing structure 88b) inside the lumen 72. Alternatively, the cutting member 34 may be disposed between the lumen 72 and the elongate body 80a (extending from the braided capture structure 88a).

During an operation, the elongate body 80 and/or the cutting member 34 can be slid, translated, and/or rotated along and within the lumens 72 to position an end of the implant capturing device 24 in close proximity to a previously deployed fixation device 10 (FIGS. 3-5). This sliding, translating, and/or rotating action can be achieved through connecting a torque handle 30 to an implant capturing device proximal end 76. The torque handle 30 allows a user to manipulate the implant capturing device 24 to position an implant capturing device distal end 88 in close proximity to a previously deployed fixation device 10 (FIGS. 3-5).

For instance, the torque handle 74 can selectively mount to a proximal end 76 of the implant removable device 24 or elongate body 80; the implant capturing device 24 is resiliently flexible and configured to transmit torque from the implant capturing device proximal end 76 to the implant capturing device distal end 88 with a predetermined torque or turning ratio, i.e., ratio of implant capturing device proximal end 76 rotation to the implant capturing device distal end 88 rotation. In some implementations, this predetermined torque or turning ratio is 1:1 (proximal turns:distal tuns), but can range from about 1:2 to about 1:5 (proximal turns:distal turns), from about 1:2 to about 1:3 (proximal turns:distal turns), from about 3:1 to about 2:1 (proximal turns:distal turns), or another torque or turning ratio.

In some configurations, the capturing structure 82 is further configured to be extended and retracted via manipulating the torque handle 74. When the capturing structure 82 identifies and centers a fixation device 10, a user can manipulate the torque handle 74 to cause the capturing structure 82 to partially retracted to cause the diameter of the distal end 88 of the capturing structure 28 to retract, which, in turn, captures and secures the fixation device 10. When the capturing structure 82 is a braided capturing structure, the retraction of distal end 88 of the braided capturing structure 82 may be retracted via tightening one or more threads of the braided capturing structure 82 via tethering back to and through the handle. Similarly, the distal end 88 of the braided capturing structure 82 may be extended via releasing the one or more threads of the braided capturing structure 82. Alternatively, or in addition, the retraction of the distal end 88 of the braided capturing structure 82 may be performed via advancing the lumen 72 toward the braided capturing structure 82 to restrict the diameter of the braided capturing structure 28 in transverse directions that are orthogonal to the longitudinal axis 48. Similarly, the distal end 88 of the braided capturing structure 82 may be released and/or extended via retracting the lumen 72 toward the proximal end 76 to release the finger conical capturing structure 82 out of the lumen 72.

When the capturing structure 82 is a finger conical capturing structure, the retraction of the distal end 88 of the finger conical capturing structure 82 may be performed via advancing the lumen 72 toward the finger conical capturing structure 82 to restrict the diameter of the finger conical capturing structure 82 in transverse directions that are orthogonal to the longitudinal axis 48. Similarly, the distal end 88 of the finger conical capturing structure 82 may be released and/or extended via retracting the lumen 72 toward the proximal end 76 to release the finger conical capturing structure 82 out of the lumen 72.

Once the capturing structure 82 captures and/or secures the fixation device 10, the capturing structure 82 may further be fully retracted into the region 28 of the elongate body 80 to withdraw the captured fixation device 10 into the region 28 where the cutting member 34 can cut the distal end of the fixation device 10 or the tissue surrounding the fixation device 10.

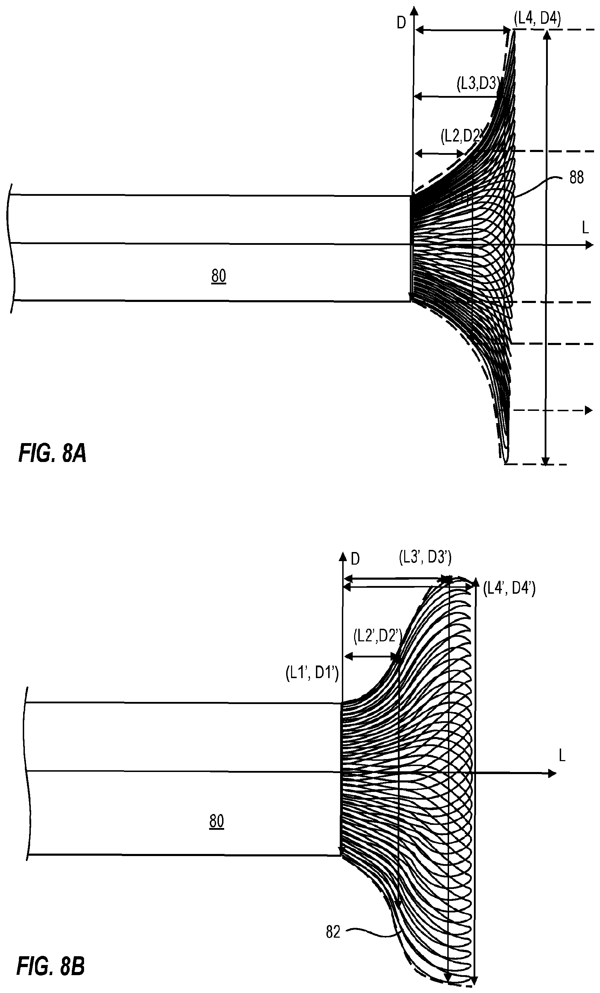

Turning to FIGS. 8A and 8B, illustrated is a configuration of the braided capturing structure 82 (corresponding to the braided capturing structure 82 of Fig. 6A) that can be extended and/or retracted. FIG. 8A illustrates that the braided capturing structure 82 is in an extended state, and FIG. 8B illustrates that the braided capturing structure 82 is in a partially retracted state. The L axis and D axis both start from the center of the proximal end of the braided capturing structure 82. The L axis points towards the dismal end in the longitudinal direction. The D axis points in a direction that is transverse to the longitudinal direction.

As illustrated in FIG. 8A, when the braided capturing structure 82 is in the extended state, the braided capturing structure 82 is generally conical or trumpet shaped. In particular, the diameter of the implant capturing structure 82 increases from the proximal end to the distal end of the implant capturing structure 82. For example, the implant capturing structure 82 has a smallest diameter D1 at its proximal end (L = L1) and has a largest diameter D4 at its distal end (where L = L4). The implant capturing structure 82at L2 in the longitudinal direction has a diameter D2, and the implant capturing structure 82at L3 in the longitudinal direction has a diameter D3. The closer to the proximal end, the smaller the diameter of the braided capturing structure 82 is; and the closer to the distal end, the larger the diameter of the braided capturing structure 82 is. As illustrated, since the point of L3 is closer to the distal end L4 than the point of L2, the diameter D3 is greater than D2.

A diameter of the capture structure 82 increases along a length of the capture structure 82. For instance, a diameter D1 may smoothly transition from an outer diameter of elongate body 80 (with or without a coating or liner), and increase to the wider end diameter D4. The increasing taper shape of capture structure 82 may be a linearly increasing ratio (example: increasing 10% in diameter for every 1mm of increasing length, but not to exceed the final D4). The diameter D4 should not exceed the inner diameter of a lumen of the sheath 100, which will be discussed in more detail hereinafter. The increasing taper shape may be non-linear, increasing at a greater ratio, e.g. 20%, 25%, 30% or some ratio or decreasing at a lesser ratio, e.g., between about 1%-10%, for a given length, and a reduced ratio for the remaining length, e.g. 5%, 4%, 3%, 2%, 1% or some other ratio less than 10%).For instance, the taper shape can be a concave or convex parabolic shape. The particular tapering map can be optimal for positioning and redirecting the distal tip of the implant into the center of the tapered capture structure. Likewise, a relative flexibility may conform to the trajectory of the implant axis if not colinear with the axis of the capture structure - aided by the engaging features of the capture structure, and by additional rotation and torque to aid in drawing the implant and tissue into the capture structure. For example, the increase of the diameter may be linear (e.g., in a strict conical-shaped structure) or non-linear (e.g., in Gabriel's horn-shaped structure). A conical shape structure may be represented as D = a*L, where "a" is a parameter that can be set based on need, L represents the longitudinal direction, and D represents the transverse direction. The greater value "a" is, the greater the diameter D increases when the length L increases. A Gabriel's horn shape may be represented as D = 1/(b*L+c), where each "b" and "c" is a parameter that can be set based on need, and similarly, L represents the longitudinal direction, D represents the transverse direction. Different trumpet curves may be achieved by adjusting "b" and "c". Other trumpet shapes may also be implemented, e.g., using equation D = 1/(d*L^{e}+f), where each of "d", "e", and "f" is a parameter that can be set based on need.

As described above, when the braided capturing structure 82 is in the extended state (FIG. 8A), the braided capturing structure 82 can provide a generally conical or trumpet-shape with a capture opening at the distal end 88 that can locate and capture the fixation device 10 (FIGS. 3-5) and tissue. When the braided capturing structure 82 is in the partially retracted state (FIG. 8B), the distal end 88 of the braided capturing structure 82 retracts to capture the fixation device 10. After that, the lumen 72 is advanced toward the captured fixation device 10. As the lumen 72 is advanced toward the captured fixation device 10, the braided capturing structure 82 can guide the fixation device 10 into the capture opening 26 so that a longitudinal axis of the fixation device 10 becomes generally aligned with a longitudinal axis of the elongate body 80.

Alternatively, or in addition, rotating the implant capturing device 24 can also help advance the braided capturing structure 82 by compressing and/or cutting into the tissue by the braided capturing structure 82. Thereafter, the distal end 88 of the capturing structure 82 is caused to retract to capture and secure the fixation device 10 (FIGS. 3-5). A fixation device that is not coaxial with the lumen 72 can be simply retrieved and positioned to approximate coaxial alignment with the lumen 72 so the fixation device 10 and tissue can be withdrawn through the opening 26 into the region 28 of the implant management tool 22 more efficiently. Stated another way, the braided capturing structure 82 is configured to selectively center a deployed implant, such as the fixation device 10, in relation to a longitudinal axis of the lumen 72 and the capture region 28 to aid with the capture and subsequent removal of the fixation device 10.

A resiliency of the braided capturing structure 82 in the partially retracted state provides a compressive force upon the fixation device and the tissue in a direction transverse to the longitudinal axis of the braided capturing structure 82. The compressive force can range from about 0.5 Newtons to about 25 Newtons.

More generally, the diameters of the braided capturing structure 82 can vary based upon an estimated size of the fixation device and any tissue to be removed with the fixation device. For instance, in one procedure a physician can measure the ingrown fixation device using fluoroscopy, intracardiac echocardiogram (ICE), three-dimensional (3D) electroanatomical mapping (EAM) systems, or other imaging modalities and select a particularly sized implant removal device. Alternatively, a single implant removal device can accommodate various sizes of ingrowth fixation device, with associated tissue, with the physician using the screw-type action to cut, penetrate, or otherwise capture the ingrowth fixation device.

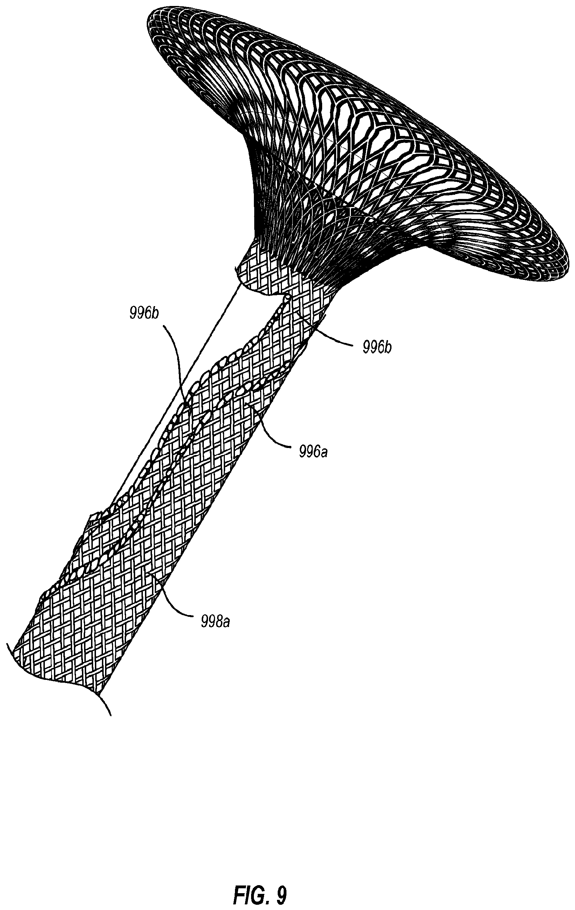

Fig. 9 illustrates an enlarged view of the braided capturing structure 82. As illustrated, the braided capturing structure 82 can include a plurality of threads that are woven together to provide one or more layers 996. In some configurations, the one or more layers 996 may be formed by folding a layer of the same braid over once or multiple times. For instance, either or both of the threads 998a, 998b may be woven in a diamond two wire two-under-two, over-two pattern; a half-load single wire over-one, one-under pattern; a full-load single wire over-two, under-two pattern; other alternating woven patterns; or combinations thereof. In other embodiments, the braid can include a single thread routed substantially straight longitudinally through the plurality of threads. The threads may be made from multiple materials, one of which could be all platinum for improving radiopacity.

In some configurations, threads 998a, 998b can be round threads, elliptical threads, or flat threads. In some configurations, the threads 998a, 998b can also have a polygonal cross-section or inclusion of features to aid frictional engagement between the braided capture device 82 and the fixation device 10 (FIGS. 3-5). The threads 998a, 998b can be made of or include a variety of reinforcement materials, such as, metals, metal alloys, thermoplastics, other polymers, or combinations thereof. In some embodiments, the reinforcement material or materials may have a greater elastic modulus than the body material. For example, the braid can include a mixture of threads 998a, 998b with different properties, such as metal or stainless-steel threads woven with polymer threads. In at least one embodiment, the braid can include a plurality of stainless-steel wires woven in a diamond pattern. Such an embodiment of the braid can include between 16 and 32 threads of stainless steel.

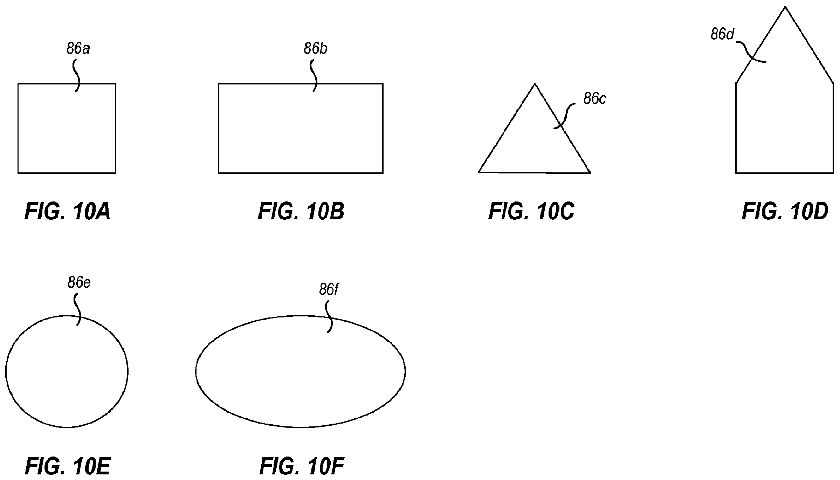

Further, to also aid with the capture of the fixation device 10 (FIGS. 3-5) and tissue retention, the braided capturing structure 82 can have a cross-sectional profile to increase contact surfaces between the braided capture structure 82 and the tissue. While a generally circular thread cross-section can be used, a polygonal cross-section or inclusion of features to aid frictional engagement between the braided capture device 82 and the fixation device 10 (FIGS. 3-5) and tissue are possible. For instance, and not by way of limitation, as illustrated in FIGS. 10A-10F, the thread of the braided capture device 82 can have cross-sections that are square, rectangular, triangular, polygonal, oval, elliptical, combinations or modifications thereof. It will also be understood that for those cross-sections having a major axis and a minor axis, either the major axis or the minor axis can be disposed to be generally parallel to or transverse to a longitudinal axis of the braided capturing structure 82 or the elongate body 80. Changing the orientation of the major axis and the minor axis can be used to vary the flexibility of the capturing structure 82 or the elongate body 80. With respect to features to increase frictional engagement, this can include projections, barbs, roughened surface finishes, coatings, rotational bias (ratcheted edges that allow for smooth rotation when rotating at least one direction-e.g. smooth clockwise rotation but tissue-engaging counter-clockwise), combinations or modifications thereof.

While discussion is made to the elongate body 80 including a lumen 72, in other configurations, the elongate body 80 can also include a hypotube, laser cut hypotubes, a braided tubular member, a laminated tube, such as Polytetrafluoroethylene(PTFE) lined braided Pebax^{®}, Pebax^{®} laminated coil, Polyimide braid or coil, thin wall carbon or Polyether ether ketone(PEEK), or combinations or modifications thereof.

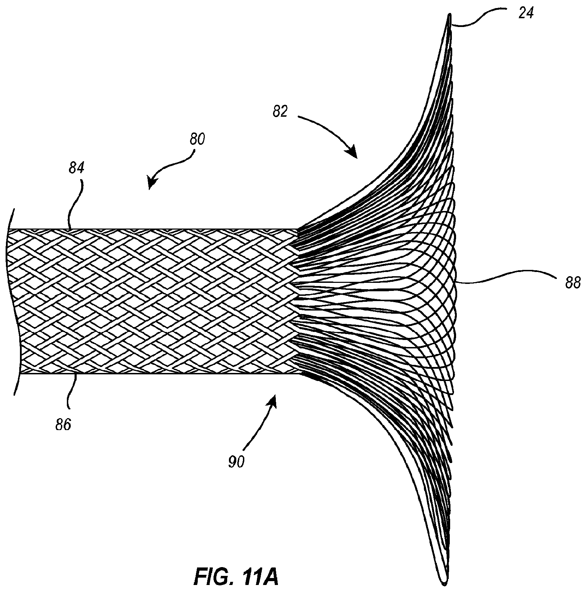

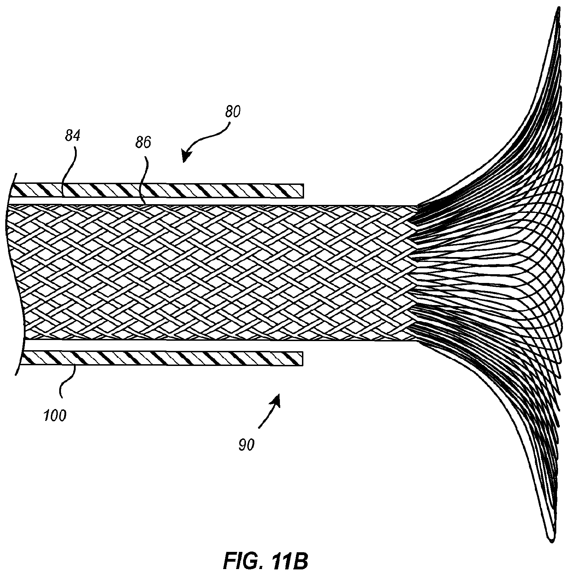

Turning to FIGS. 11A and 11B, illustrated are configurations of the implant removal device 80, with FIG. 11B illustrating the implant removal device 80 with an optional sheath 100. As illustrated, the implant removal device 24 includes a braided member 84 that extends along both the elongated body 80 and the capturing structure 82. In some configurations, the braided member 84 is flexible in bending and provides little or no elongation in either compression or tension. For instance, the braided member 84 along the elongate body 80 is tightly braided, where adjacent threads contact each other, along the length of the elongate body 80. With adjacent thread contacting each other, there is no elongation in compression of the elongate body 80 during distal movement. In tension, such as when the elongate body 80 is withdrawn proximally, the braided member 86 remains substantially in contact with each other.

Alternatively, in tension, there can be a small amount of separation so that any elongation of the elongate body 80 is between about 5 percent and about 30 percent of the length of the elongate body. With such elongation, a physician can manipulate and position the implant removal device 24 with the patient's anatomy to capture the fixation device 10 (FIGS. 3-5) and tissue.

In contrast to the elongate body 80, the threads of the braided member 84 forming the capturing structure 82 may have a constant or variable amount of separation from a transition or junction 90 between the capturing structure 82 and the braided member 84 toward the distal end 24 of the implant removal device 24.

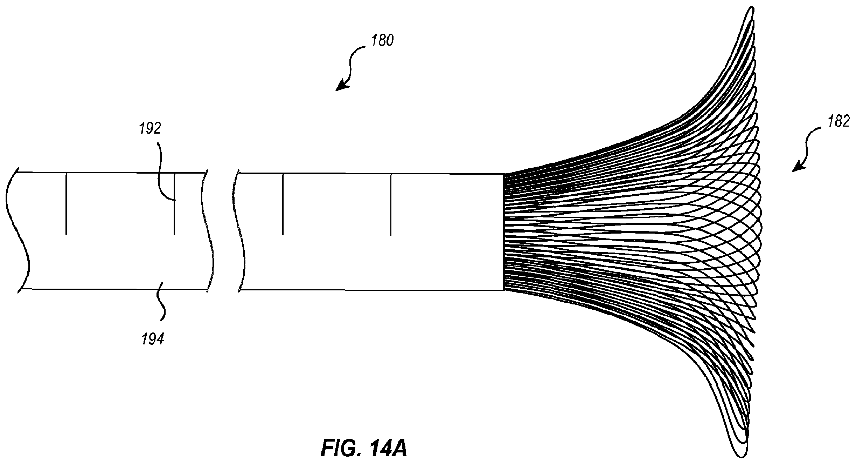

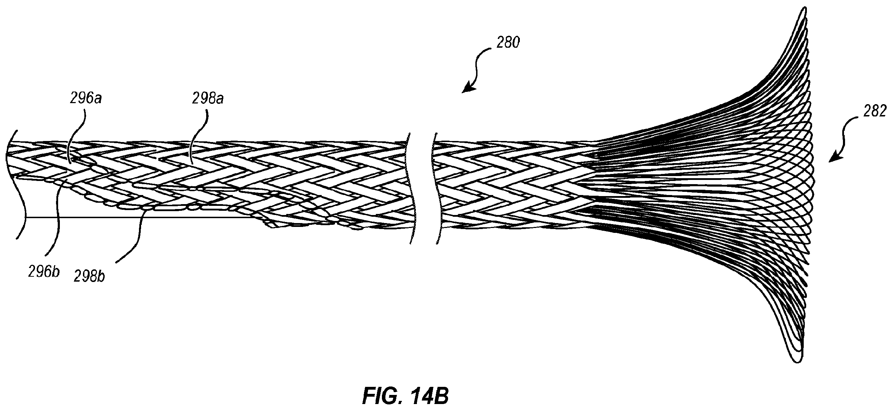

Referring to FIG. 11B, in some embodiments, the braided member 84 is optionally covered with a sheath 100, which will be further discussed in more detail later with respect to FIGS. 14A and 14B.

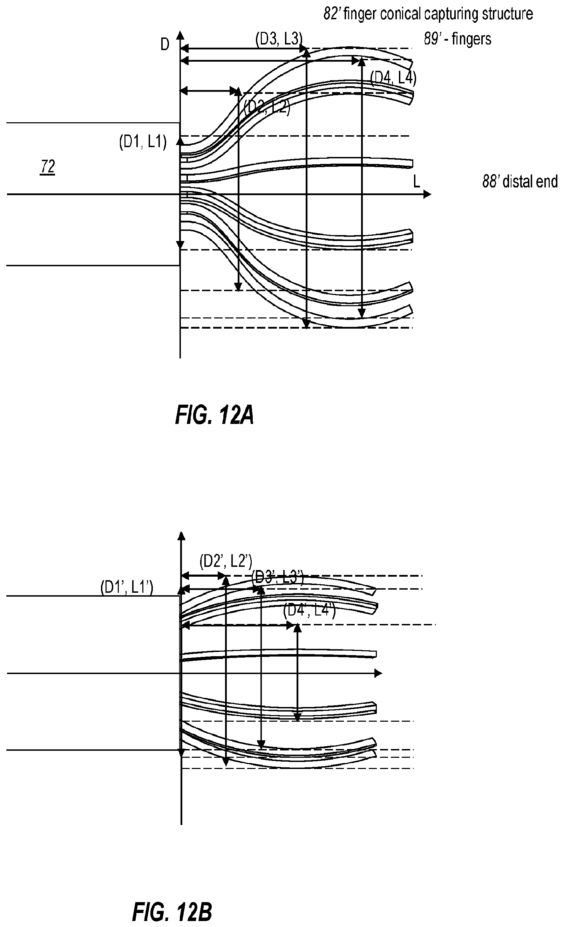

Turning to FIGS. 12A and 12B, illustrated is a configuration of the finger conical capturing structure 82' (corresponding to the finger conical capturing structure 82 of FIG. 6B) that can be extended and/or retracted. FIG. 12A illustrates that the finger conical capturing structure 82' is in an extended state, and FIG. 12B illustrates that the finger conical capturing structure 82' is in a partially retracted state. The L axis and D axis both start from the center of the proximal end of the finger conical capturing structure 82'. The L axis points towards the dismal end in the longitudinal direction. The D axis points in a direction that is transverse to the longitudinal direction.

As illustrated in FIG. 12A, when the finger conical capturing structure 82' is in the extended state, the finger conical capturing structure 82' looks like a relaxed hand having a plurality of fingers 89', extending out from the distal end of the lumen 72. The diameter of the finger conical capturing structure 82' is restricted by the distal end of the lumen 72. Thus, the diameter D1 at the distal end of the lumen 72 is the smallest. Similar to a relaxed hand, the diameter of the fingers increases in the L direction gradually from D1 to D2 and D3, then reduces near the distal end 88' of the finger conical capturing structure 82'. For example, at the distal end L4, the diameter of the finger conical capturing structure 82' reduces to D4 < D3, where L4>L3.

As illustrated in FIG. 12B, when finger conical capturing structure 82' is in the partially retracted state, the lumen 72 advances toward the distal end 88 of the finger conical capturing structure 82' to cause the finger conical capturing structure 82' to be partially retracted into the lumen 72. The lumen 72 restricts the fingers 89' in the transverse direction D to cause the diameter of the not yet retracted portion of the fingers to reduce. As illustrated, the diameter of the fingers 89' may expand slightly outside the lumen, then reduces sharply. The tips of the fingers 89' (i.e., the distal end of the finger conical capturing structure 82') have the smallest diameter D4, depending on the relative position of the distal end 88 and the lumen 72, and/or the size of the fixation device 10 and/or the surrounding tissue that is captured by the fingers 89'. In such a partially retracted state, when there is no object captured within the fingers 89', the natural diameter of the fingers 89' may be 0; and when an object is captured by the fingers 89', the finger conical capturing structure 82' looks like a hand (with a plurality of fingers 89') holding the object.

As illustrated, in the extended state, the finger conical capturing structure 82' can provide a generally circular opening at the distal end 88' that can locate and capture the fixation device 10 (FIGS. 3-5) and the surrounding tissue. As the lumen 72 is advanced toward the captured fixation device 10, distal end 88' of the finger conical capturing structure 82' retracts to capture the fixation device 10. After that, the lumen 72 is further advanced toward the captured fixation device 10, while the finger conical capturing structure 82' guides the fixation device 10 into the capture opening 26 so that a longitudinal axis of the fixation device 10 becomes generally aligned with a longitudinal axis of the lumen 72. Alternatively, or in addition, in some configurations, rotating the implant capturing device 24 can also help advance the finger conical capturing structure 82' via compressing or penetrating the tissue by the fingers 89' of the finger conical capturing structure 82'.

Similar to the braided capturing structure 82, the finger conical capturing structure 82' is also configured to selectively center a deployed implant, such as the fixation device 10, in relation to a longitudinal axis of the elongate body 80 and the capture region 28 to aid with the capture and subsequent removal of the fixation device. For example, a fixation device 10 that is not coaxial with the elongate body 80 and/or the finger conical capturing structure 82' can be simply retrieved and positioned to approximate coaxial alignment with the elongate body 80 so the fixation device 10 an tissue can be withdrawn through the opening 26 into the region 28 of the implant management tool 22 more efficiently.

Also similar to the braided capturing structure 82, a resiliency of the finger conical capturing structure 82' in the partially retracted state provides a compressive force upon the fixation device and the tissue in a direction transverse to the longitudinal axis of the braided capturing structure 82. The compressive force can range from about 0.5 Newtons to about 25 Newtons.

Additionally, similar to the threads of the braided capturing structure 82, to aid with the capture of the fixation device 10 (FIGS. 3-5) and tissue retention, the fingers 89' can have a cross-sectional profile to increase contact surfaces between the fingers 89 of the finger conical capturing structure 82' and the tissue. While a generally circular wire can be used, a polygonal cross-section or inclusion of features to aid frictional engagement between the fingers 89 and the fixation device 10 and tissues are possible. For instance, and not by way of limitation, as illustrated in FIGS. 10A-10F, the fingers 89' of the finger conical capturing structure 82' can have cross-sections that are square, rectangular, triangular, polygonal, oval, elliptical, combinations or modifications thereof. It will also be understood that for those cross-sections having a major axis and a minor axis, either the major axis or the minor axis can be disposed to be generally parallel to or transverse to a longitudinal axis of the finger conical capturing structure 82' or the elongate body 80. Changing the orientation of the major axis and the minor axis can be used to vary the flexibility of the capturing structure 82 or the elongate body 80. With respect to features to increase frictional engagement, this can include projections, barbs, roughened surface finishes, coatings, rotational bias (ratcheted edges that allow for smooth rotation when rotating at least one direction-e.g. smooth clockwise rotation but tissue-engaging counter-clockwise), combinations or modifications thereof.

Again, similar to the braided capturing structure 82, while discussion is made to the elongate body 80' including a lumen 72, in other configurations, the elongate body 80 can also include a hypotube, laser cut hypotubes, a braided tubular member, a laminated tube, such as Polytetrafluoroethylene(PTFE) lined braided Pebax^{®}, Pebax^{®} laminated coil, Polyimide braid or coil, thin wall carbon or Polyether ether ketone(PEEK), or combinations or modifications thereof.

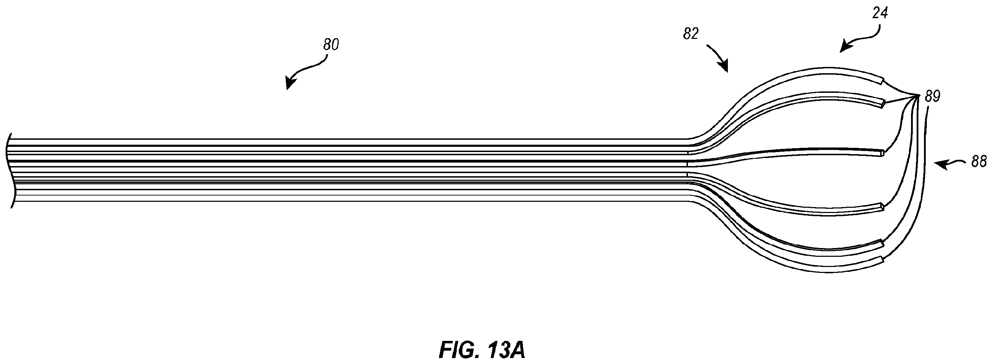

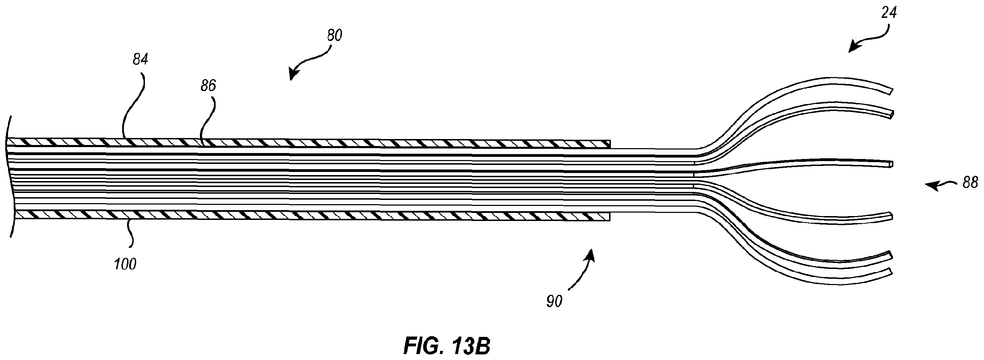

Turning to FIGS. 13A and 13B, illustrated are configurations of the implant removal device having an elongate body 80' and a finger conical capturing structure 82', with FIG. 13B illustrating that elongate body 80' includes an optional sheath 100. As illustrated, the implant removal device 24 includes a plurality of wires 89' that extends through both the elongate body 80 and the finger conical capturing structure 82'. In some embodiments, the proximal end of the wires 89' may be braided or bound together, such that the extending and retracting of all the fingers 89' are controlled simultaneously. Alternatively, the wires 89' are not bound together, such that each wire 89' may be individually controlled. Alternatively, or in addition, a ring structure is disposed at a proximal end of the elongate body 80 with the wires terminating at the ring structure.

Each wire 89' in the elongate body 80' may be curved in transverse directions (e.g., curved from a center longitudinal axis 48 towards the outer area of the elongate body 80), such that when the wire 89 extends out of the elongate body 80', the extended portion of the wire 89' expands in the transverse directions. At the same time, the tip (i.e., distal) end of the wires 89' may be curved inwardly toward the center longitudinal axis 48 to cause the wires 89' to form the finger conical shape.

Referring to FIG. 13B, in some embodiments, the wires 89' are covered with an optional sheath 100', which is similar to the optional sheath 100 of FIG. 11B. More details related to the optional sheath 100 or 100' will be discussed now with respect to FIGS. 14A and 14B. FIGS. 14A and 14B illustrate example configurations of sheath 100 of FIG. 11B and/or sheath 100' of FIG. 13B. Note, the optional sheath 100 or 100' may generally go over the braided member 86 or wires of fingers 89', but need not be integrated with the wires of the braided member 86 or the wires of the fingers 89', such that the braided member 86 or the fingers 89' can be contained in the sheath, thereby reducing the delivery diameter of the device. For simplicity, like reference numerals are associated with like structures and the discussion of other implant removal devices herein is also applicable to this configuration.

As illustrated in FIG. 14A, an alternate configuration of the implant removal device having a hypotube sheath is illustrated. When the elongate body includes a hypotube 180, the hypotube 180 can include a plurality cuts or slits 182 that control a flexibility of the elongate body 180. The cuts or slits 182 can be elongate, form through holes or island cuts, slits, zig-zags, or combinations or modifications thereof. The cuts or slits 182 can be linear, diamond-shaped, square, rhombohedral, triangular, rectangular, circular, oblong, other elliptical or oval shapes, other regular or irregular polygonal shapes, or modification or combinations thereof.

The cuts or slits 182 can form at least one longitudinally continuous spine 194 that can preferably be continuous and uninterrupted along a longitudinal length of, and located at a fixed angular location on, the elongate body 180. Having a longitudinally continuous spine 194 allows the elongate body 180 to transmit tension force applied at the elongate body proximal end to the elongate body distal end without substantial elongation of the elongate body 180. In other embodiments, the longitudinally continuous spine 194 can may allow the elongate body 180 to transmit compression force applied at the elongate body proximal end to the elongate body distal end without substantial shortening of the elongate body. For example, the compressive force can range from about 0.9 Newtons to about 1.3 Newtons, from about 1.1 Newtons to about 1.5 Newtons, from about 2.2 Newtons to about 4.4 Newtons, from about 4.4 Newtons to about 6.6 Newtons.

As illustrated in FIG. 14B, an alternate configuration of the implant removal device is illustrated. When the elongate body include a braided tubular sheath 280 it can include a braid configuration that prevents unwanted compression during distal translation and unwanted elongation under tension during proximal translation of the elongate body 180 during positioning of the capturing structure 282. For instance, the braid can include a plurality of threads that are woven together to provide one or more layers 296. In some configurations, the one or more layers 996 may be formed by folding a layer of braided material once or multiple times. For instance, either or both of the threads 298a, 298b may be woven in a diamond two wire two-under-two, over-two pattern; a half-load single wire over-one, one-under pattern; a full-load single wire over-two, under-two pattern; other alternating woven patterns; or combinations thereof. In other embodiments, braid can include a single thread routed substantially straight longitudinally through the plurality of threads.

The threads 298a, 298b can be round threads, elliptical threads, or flat threads. The threads 298a, 298b can be made of or include a variety of reinforcement materials, such as, metals, metal alloys, thermoplastics, other polymers, or combinations thereof. In some embodiments, the reinforcement material or materials may have a greater elastic modulus than the body material. For example, the braid can include a mixture of threads 298a, 298b with different properties, such as metal or stainless-steel threads woven with polymer threads. In at least one embodiment, the braid can include a plurality of 304 stainless-steel wires woven in a diamond pattern. Such an embodiment of the braid can include between 16 and 32 threads of stainless steel.

Generally, the implant capturing device 24 (including the elongate body 80, the capturing structure 28, the lumen 72 and/or the optional sheath 100, 100') can be fabricated from one or more of various materials, such as metals, alloys, polymers, ceramics, shape memory materials including shape member alloys or shape member polymers, superelastic materials, Polytetrafluoroethylene(PTFE), Pebax^{®}, thin wall carbon materials, Polyether ether ketone(PEEK), combinations thereof or modifications thereto. Additionally, the material forming the implant removable device 24 can be processed to achieve differing flexibilities along the length of the implant capturing device 24. For instance, the junction 90 between the elongate body 80 and the capturing structure 82, 82' can be cold worked to increase or decrease the stiffness to change the flexibility characteristics or properties. Providing decreased stiffness, increasing flexibility, allows the braided capturing structure 82 to locate the fixation device more easily. Instead of cold working, it will be understood that adjacent threads of the capturing structure 82, 82' can be welded or otherwise bonded together, thereby increasing a stiffness of the implant capturing device 24 at the location of the welding or bonding

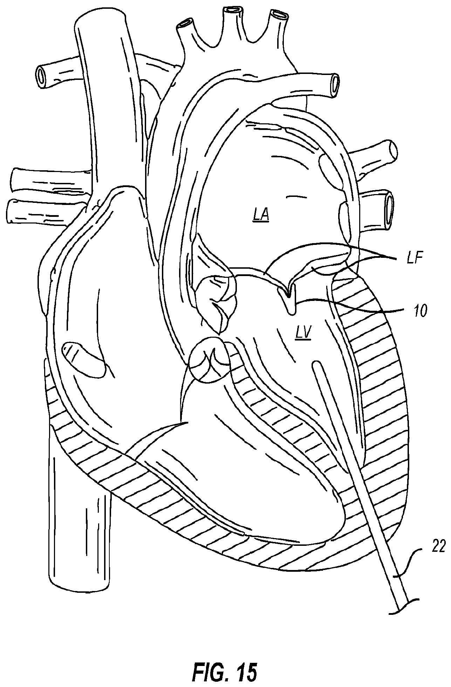

Referring now to FIG. 15, illustrated is a cut-away view of a heart with a portion of an implant management tool 22 according to the present invention and accessing the heart using an apical approach. It is contemplated that other surgical approaches to the heart, and valves in addition to the mitral valve, are within the scope of the inventive subject matter claimed herein. FIG. 15 shows a portion of the implant management tool 22 advanced toward mitral valve having a previously deployed fixation device, such as the fixation device 10 (FIGS. 3-5).

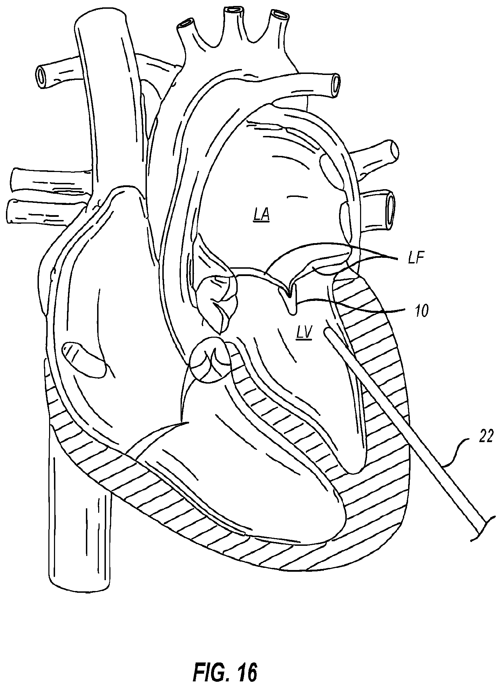

FIG. 16 shows the lateral deployment of one embodiment of a portion of an implant management tool 22 according to the present invention and shows a portion of an implant management tool positioned within the left ventricle towards the mitral valve by way of a lateral trans-ventricular wall approach through the lateral wall of the left ventricle of the heart.

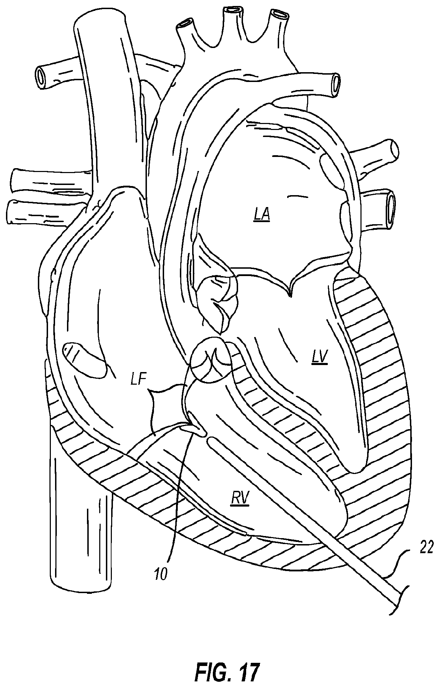

FIG. 17 is a cut-away view of a heart with a portion of an implant management tool 22 according to the present invention and accessing the heart using an apical approach into the right ventricle. It is contemplated that other surgical approaches to the heart, and valves in addition to the mitral valve, are within the scope of the inventive subject matter claimed herein. FIG. 17 shows the portion of an implant management tool advanced toward the tricuspid valve.

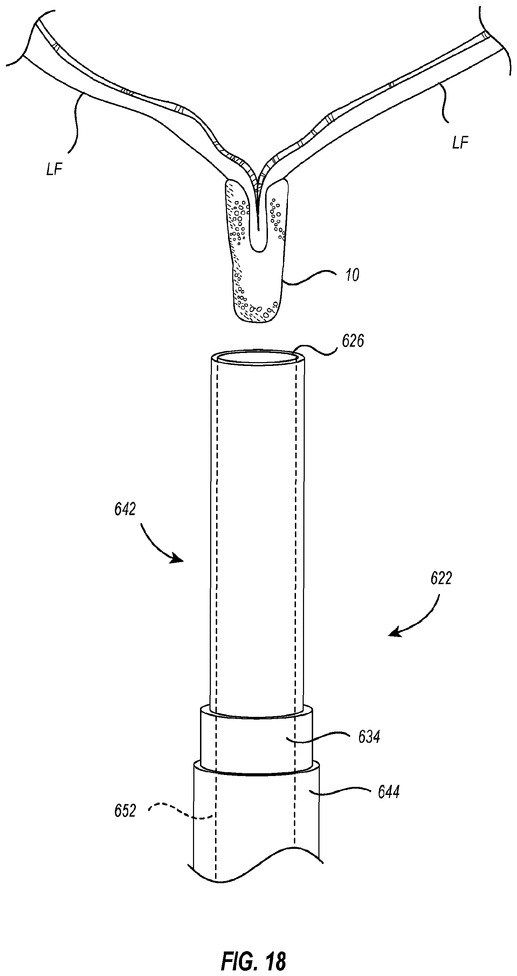

FIGS. 18-22 are a series of drawings illustrating the deployment of the implant management tool and the implant removal device and subsequent capture and removal of a previously deployed fixation device, such as the fixation device 10 (FIGS. 3-5). For simplicity, FIGS. 18-22 illustrate a portion of the anatomy from FIGS. 15-17 and a portion of each of the implant management tool and the implant removal device.

As referenced previously in the configuration of FIG. 6A, with the opening 26 being at the dismal end of the shaft 44, a greater space can be provided to capture the fixation device 10 (FIGS. 3-5) and tissue than if the opening were, for example, coaxial with the lumen of the shaft. FIG. 18 illustrates implant management tool 622 with an opening 626 formed in a distal end 642, with a cutting member 634 coaxial with the lumen 652. The operation and use of the implant management tool 622 is similar to that of the implant management tool 22 except that the fixation device is drawn into the lumen 652 through the opening 626 at a distal-most end of a shaft 644.

FIGS. 18-22 are a series of drawings illustrating the deployment of the implant management tool and the implant removal device and subsequent capture and removal of a previously deployed fixation device, such as the fixation device 10 (FIGS. 3-5). For simplicity, FIGS. 18-22 illustrate a portion of the anatomy from FIGS. 15-17 and a portion of each of the implant management tool and the implant removal device.

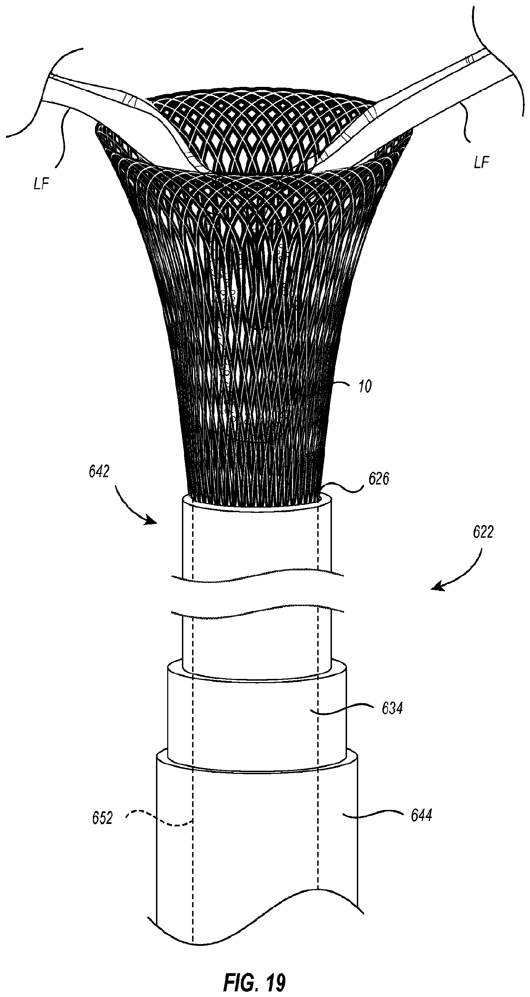

As illustrated in FIG. 19, the implant management tool 622 is advanced toward the previously deployed fixation device 10. With the opening 626 being generally orientated toward the deployed fixation device 10, the implant removal tool 24 can be translated or moved along the lumen 652 so the capturing structure 82 is advanced toward the deployed fixation device 10. This can be achieved, with reference to FIG. 6A, by manipulating the proximal end 40 of the implant removal tool 24 either directly or by way of the torque handle 74, as described in relation to FIG. 6A.

A user can further manipulate the proximal end 40 (FIG. 6A) to advance the capturing structure 82 over the fixation device 10, as illustrated in FIG. 19. As the user advances the capturing structure 82 towards the fixation device 10, such as by torqueing the elongate body 80 to rotate the capturing structure 82 and advance the capturing structure 82 along a portion of tissue surrounding the fixation device 10, the conical or trumpet shape of the capturing structure 82 begins to axially align the fixation device 10 with a longitudinal axis of the elongate body 80. For instance, as the implant removal tool 24 is rotated, the capture device 82 engage with the tissue, applying a compressive force against the tissue and the fixation device 10 and draw the fixation device 10 and tissue into the capture opening 26. Optionally, the distal end 88 of the capturing structure 82 can penetrate the tissue to provide enhanced capturing of the previously deployed fixation device 10 through both compressive force and mechanical engagement with the tissue.

When the capturing structure 82 has been advanced sufficiently along the length of the fixation device 10, such that the distal end of the capturing structure 82 is positioned at close to or past an end of the fixation device 10 closest to the leaflets, a physician or clinician can verify its location by fluoroscopy, intracardiac echocardiogram (ICE), or three-dimensional (3D) electroanatomical mapping (EAM) systems. Because the capturing structure 82 is formed of a radiopaque material, the physician or clinician can verify the location with relative ease.

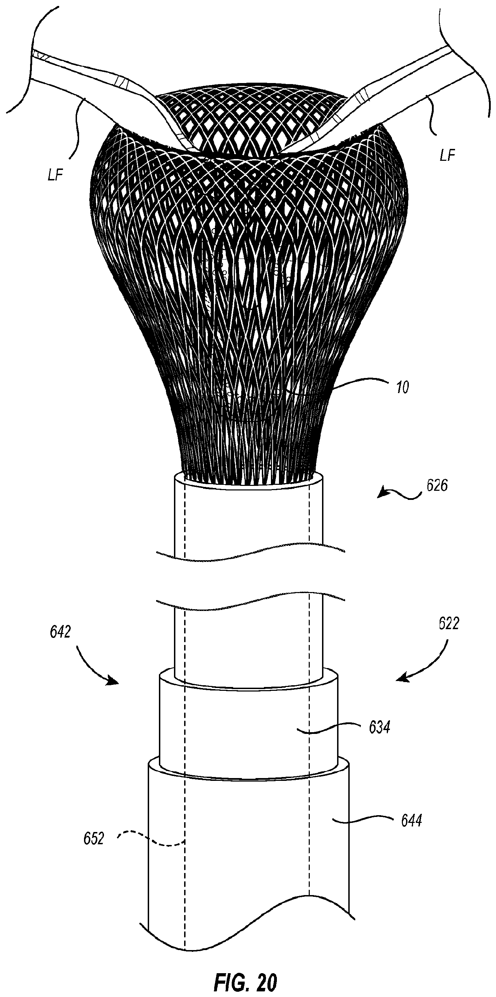

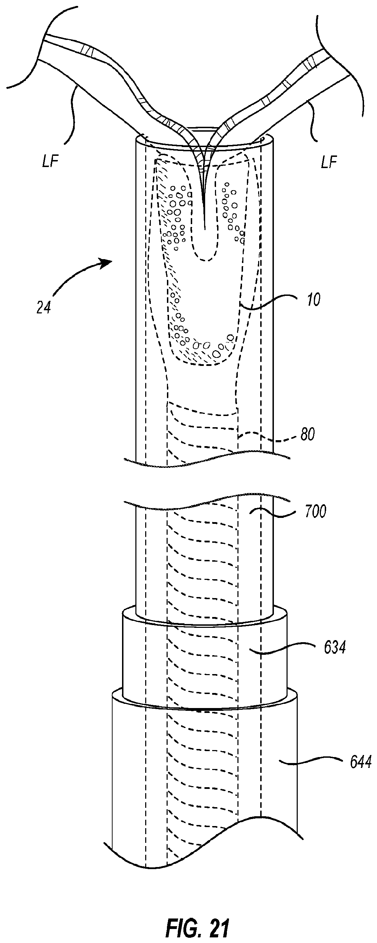

Once verified, the distal end 88 of the capturing structure 82 is retracted to capture or secure the fixation device 10, as illustrated in Fig. 20. Thereafter, the capturing device 24 is drawn proximally to apply tension to the capturing structure 82, as illustrated in FIG. 21 (the capturing structure 82 being illustrated in a simpler form for clarity). This tension can at least partially increase a length of the capturing structure 82, including increasing a compressive force on the fixation device 10 and the tissue. The compressive force can be a transverse force that is applied transversely to a longitudinal axis of the capturing structure 82.

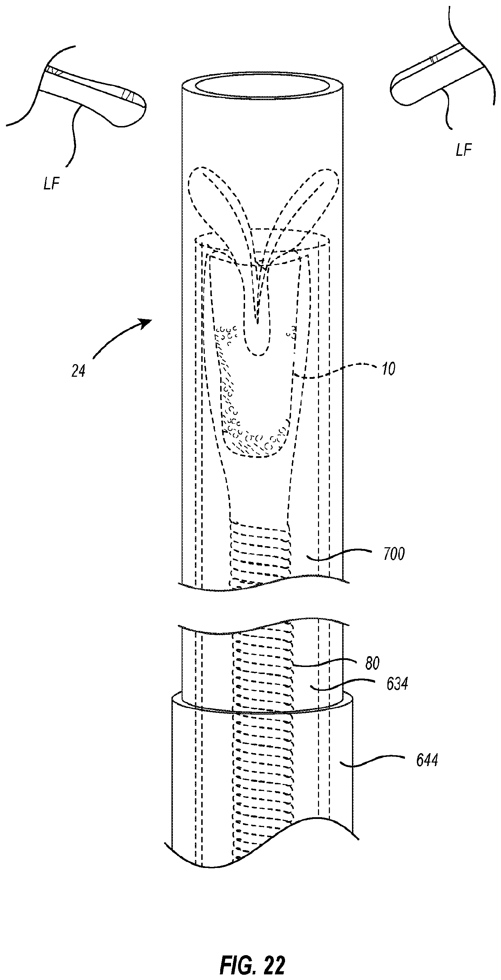

With the tissue partially caught, the sheath 700, such as sheath 100 from FIG. 11B, can be advanced over the capture fixation device 10, as illustrated in FIG. 21. With the fixation device 10 restrained with the sheath 700 manipulation of the actuator 32 of the handle 30 can translate, and optionally rotate, the cutting member 634 to at least cut or separate the fixation device 10 from the tissue surrounding the fixation device 10, as illustrated in FIG. 22 (the capturing structure 82 being illustrated in a simpler form for clarity). For instance, the actuator 32 advances the cutting member 634 with an annular cutting edge to cut the tissue. The detached fixation device 10 is retained within the lumen 652. Once the fixation device 10 is detached from the surrounding tissue, the implant removal system, with the captured fixation device 10 can be removed from the patient.

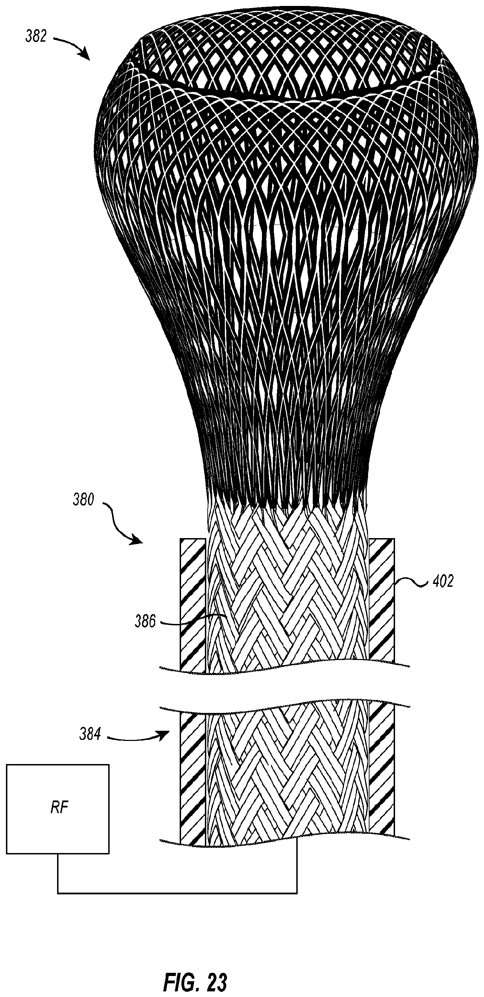

Turning to FIG. 23, illustrated is an alternate embodiment of the capturing structure according to the present invention. For simplicity, like reference numerals are associated with like structures and the discussion of other implant removal devices herein is also applicable to this configuration. As mentioned previously, a radiofrequency (RF) or an ultrasonic cutting arrangement can be used instead of the cutting member. The shape of the RF cutting element or the ultrasonic cutting element can adopt any of the above-described configurations.

In the illustrated configuration of FIG. 23, the implant removal device 380 is configured to include an RF cutting arrangement, thereby eliminating the need for the cutting member of the implant management tool and, in some configurations, the implant management tool as a whole. For instance, a catheter can be used to access the previously deployed fixation device, such as using an apical, transfemoral, trans-jugular, trans-radial, or other approach, and once the catheter is positioned, such as advancing the catheter a previously deployed guidewire, advancing the implant removal device 380 along the catheter to the previously deployed fixation device. This simplifies the implant management tool and provides an implant removal device that both cuts and cauterizes the leaflets during fixation device removal.

As illustrated, the capturing structure 382 can be utilize a bipolar or monopolar technique to delivery RF energy to the tissue. The capturing structure 382 can include a conductive portion 400 and an insulative portion 402 that is closer to the elongate body 380. For instance, braided member 386 forms the conductive portion 400 acting as an RF electrode, while the remainder of the braided member 386 are insulated. A proximal end of the implant removal device 324 is electrically connected to an RF generator 404 that provides the RF energy to the conductive portion 400. Once the capturing structure 382 is positioned around at least a portion of the fixation device, and tissue, and optionally tensioned as discussed above, activation of the RF generator 404 delivers RF energy to the tissue, thereby separating or cutting and allowing withdrawing of the extended fixation device and capturing within the catheter.

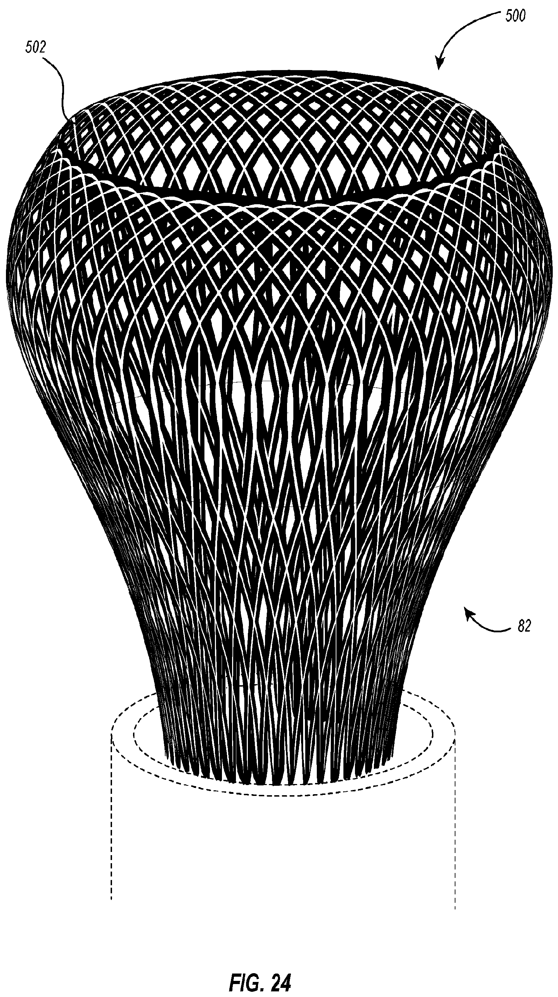

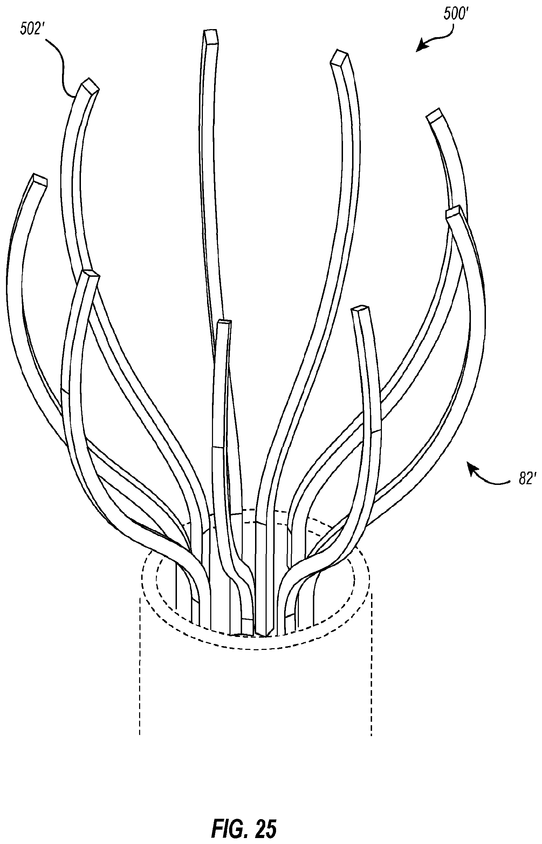

Turning to FIGS. 24-26, illustrated are alternate embodiments of the implant removal device 480 according to the present invention. For simplicity, like reference numerals are associated with like structures and the discussion of other implant removal devices herein is also applicable to this configuration.

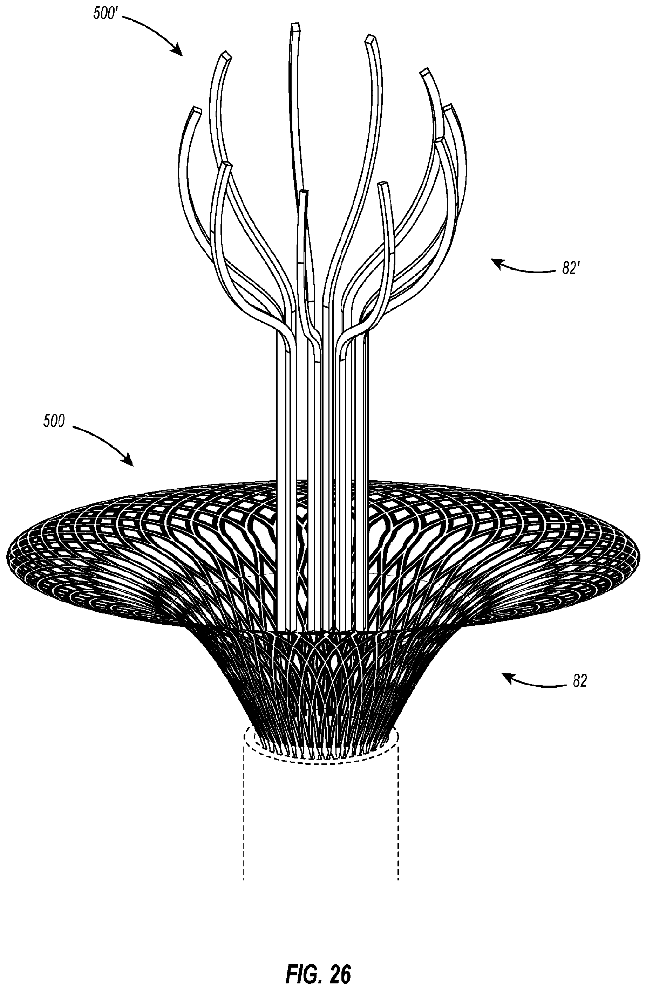

As mentioned previously, and referring to FIGS. 24-26, the implant removal device can include optional cutting surfaces 500 or 500'. The cutting surfaces 500, 500' can be sharpened edges 502, 502', such as at the corners or surfaces of the distal end 88 of the braided capturing structure 82 or the tips of the finger conical capturing structure 82'. A user can manipulate torque handle 74 to rotate and retract the braided capturing structure 82 and/or the finger conical capturing structure 82' to cut the tissue surrounding the fixation device 10. Notably, as illustrated in FIG. 26, a combination of a braided capture structure 82 and a finger conical capture structure 82' is implemented. In particular, the finger conical capturing structure 82' is disposed within the braided capturing structure 82'. In some embodiments of FIG. 26, both the surface 500' of the finger conical capture structure 82' and the surface 500 of the braided capture structure 82 may be sharpened, and each of the sharpened surfaces 500, 500' is configured to rotate to cut simultaneously or separately. Alternatively, only one of the surfaces 500 or 500' is sharpened. While the discussion was provided of the distal end 88, 88' that can penetrate tissue in the configuration of FIGS. 18-22, in some configurations, more than one cutting surfaces 500, 500' may be formed on the braided capturing structure 82 or the finger conical capturing structure 82'.

Note, the cutting surface 500 is not necessarily a continuous surface, the cutting surfaces 500 can be discrete or non-continuous, such as one more projections, barbs, etc. that can be uniformly or non-uniformly distributed or arranged in a particular manner, and optionally having different sizes and shapes. With the optional cutting surfaces, such as cutting surface 500, torqueing the elongate body rotates the capturing structure and cuts or separates tissue surrounding the fixation device 10.

The articles "a," "an," and "the" are intended to mean that there are one or more of the elements in the preceding descriptions. The terms "comprising," "including," and "having" are intended to be inclusive and mean that there may be additional elements other than the listed elements. Additionally, it should be understood that references to "one embodiment" or "an embodiment" of the present disclosure are not intended to be interpreted as excluding the existence of additional embodiments that also incorporate the recited features. Numbers, percentages, ratios, or other values stated herein are intended to include that value, and also other values that are "about" or "approximately" the stated value, as would be appreciated by one of ordinary skill in the art encompassed by embodiments of the present disclosure. A stated value should therefore be interpreted broadly enough to encompass values that are at least close enough to the stated value to perform a desired function or achieve a desired result. The stated values include at least the variation to be expected in a suitable manufacturing or production process, and may include values that are within 5%, within 1%, within 0.1%, or within 0.01% of a stated value.

A person having ordinary skill in the art should realize in view of the present disclosure that equivalent constructions do not depart from the spirit and scope of the present disclosure, and that various changes, substitutions, and alterations may be made to embodiments disclosed herein without departing from the spirit and scope of the present disclosure. Equivalent constructions, including functional "means-plus-function" clauses are intended to cover the structures described herein as performing the recited function, including both structural equivalents that operate in the same manner, and equivalent structures that provide the same function. It is the express intention of the applicant not to invoke means-plus-function or other functional claiming for any claim except for those in which the words' means for' appear together with an associated function. Each addition, deletion, and modification to the embodiments that falls within the meaning and scope of the claims is to be embraced by the claims.

The terms "approximately," "about," and "substantially" as used herein represent an amount close to the stated amount that still performs a desired function or achieves a desired result. For example, the terms "approximately," "about," and "substantially" may refer to an amount that is within less than 5% of, within less than 1% of, within less than 0.1% of, and within less than 0.01% of a stated amount. Further, it should be understood that any directions or reference frames in the preceding description are merely relative directions or movements. For example, any references to "up" and "down" or "above" or "below" are merely descriptive of the relative position or movement of the related elements.

Following are some further example embodiments of the invention. These are presented only by way of example and are not intended to limit the scope of the invention in any way. Further, any example embodiment can be combined with one or more of the example embodiments.

Embodiment 1. An implant removal device including an elongate body and a capturing structure. The elongate body has a proximal end and a distal end. The elongate body is resiliently flexible and configured to transmit torque from the proximal end to the distal end with a predetermined turning ratio. The capturing structure extends distally from the distal end and has a capture region. The capturing structure is configured to selectively center a deployed implant in relation to a longitudinal axis of the elongate body and enclose the deployed implant.

Embodiment 2. The implant removal device of embodiment 1, wherein the capturing structure includes a braided conical structure having a trumpet curved convex shape or a straight conical shape.

Embodiment 3. The implant removal device of any of embodiment 1, wherein the capturing structure includes a finger conical structure.

Embodiment 4. The implant removal device of any of embodiments 1 and 3, wherein an angular orientation of a distal end of a finger of the finger conical structure is about 0 degree to 45 degrees in relation to the longitudinal axis.

Embodiment 5. The implant removal device of any of embodiments 1 and 3-4, wherein a portion of a finger of the finger conical structure has a radius of curvature of about 5 mm to about 50 mm in a deployed/pre-capture state.

Embodiment 6. The implant removal device of any of embodiments 1-5,wherein the capturing structure includes a double-wall conformable structure.

Embodiment 7. The implant removal device of any of embodiments 1-6, wherein the capturing structure includes a shape-memory material.

Embodiment 8. The implant removal device of any of embodiments 1-7, wherein a distal end of the capturing structure has a cross-sectional major axis, in a direction transverse to a longitudinal axis of the elongate body, of about 1.5 times to about 15 times larger than a cross-sectional major axis of the proximal end.

Embodiment 9. The implant removal device of any of embodiments 1-8, wherein the elongate body comprises a hypotube with a plurality of cuts that control a flexibility of the elongate body.

Embodiment 10. The implant removal device of any of embodiments 1-9, wherein the capturing structure is configured to apply a transverse force of about 0.5 Newtons to about 25 Newtons to the implant captured by the conical capturing structure.

Embodiment 11. An implant removal device includes an implant management tool and an implant capturing device. The implant management tool is configured for use in selectively separating an implant from tissue to which the implant is attached. The implant management tool includes a distal opening with a cutting member configured to selectively extend from the distal opening to cut the tissue. The implant capturing device selectively extends from the distal end of the implant management tool. The implant capturing device includes an elongate body and a capturing structure. The elongate body has a proximal end and a distal end. The elongate body is resiliently flexible. The capturing structure extends distally from the distal end of the elongate body and has a capturing region. The capturing structure is configured to selectively center a deployed implant in relation to a longitudinal axis of the elongate body and encloses the employed implant.

Embodiment 12. The implant removal device of any of embodiment 11, wherein the implant management tool includes a handle and a shaft extending distally from the handle.

Embodiment 13. The implant removal device of any of embodiments 11-12, wherein the cutting member is disposed within the shaft.

Embodiment 14. The implant removal device of any of embodiments 11-13, wherein the implant capturing device is slidably disposed within a lumen of the cutting member.

Embodiment 15. The implant removal device of any of embodiments 11-14, wherein the cutting member is configured to slide along the implant capturing device and collapse the capturing structure.

Embodiment 16. The implant removal device of any of embodiments 11-15, wherein the capturing structure includes a braided conical structure having a trumpet curved convex shape or a straight conical shape.

Embodiment 17. The implant removal device of any of embodiments 11-16, wherein the capturing structure includes a finger conical structure.

Embodiment 18. The implant removal device of any of embodiments 1-17, wherein the cutting member includes a cutting edge that extends at least partially around a circumferential edge of the distal end of the cutting member.

Embodiment 19. The implant removal device of any of embodiments 1-18, wherein the implant removal device comprises an implant removal device from any one of claims 1-10.

Embodiment 20. A method of removing an implant includes advancing an implant removal device towards an implant deployed on tissue. The implant removable device includes an elongate body and a braided capturing structure. The elongate body has a proximal end and a distal end. The elongate body is resiliently flexible and configured to transmit torque from the proximal end to the distal end with a predetermined turning ratio. The braided capturing structure extends distally from the distal end and has a capture region. The braided capturing structure is configured to selectively center a deployed implant in relation to a longitudinal axis of the elongate body and enclose the deployed implant. The method further includes capturing the implant with the braided capturing structure and removing the implant from the patient.

Embodiment 21. The method of embodiment 20, further including positioning an implant management tool toward the tissue.

Embodiment 22. The method of embodiment 20-21, wherein capturing the implant further includes torquing the elongate body to rotate the capturing structure and advance the capturing structure along a portion of tissue surrounding the implant.

Embodiment 23. The method of embodiments 20-22, wherein the portion of the tissue surrounding the tissue is ingrowth tissue encapsulating the implant.

Embodiment 24. The method of embodiments 20-23, wherein capturing the implant further includes advancing a cutting member over the braided capturing structure to collapse the braided capturing structure.

Embodiment 25. The method of embodiments 20-24, wherein the capturing structure includes a braided structure.

Embodiment 26. The method of embodiments 20-24, wherein the capturing structure includes a plurality of fingers.

Embodiment 27. The method of embodiments 20-26, further includes separating the implant from the tissue.

Embodiment 28. The method of embodiments 20-27, wherein the tissue is a valve leaflet.

Embodiments 29. The method of embodiments 20-24, and 27-28, wherein the capturing structure includes a braided capturing structure with a finger capturing structure disposed within the braided capturing structure.

Embodiments 30. The method of embodiments 20-29, further including advancing a cutting member with the capturing structure to collapse the finger capturing structure.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.
Further aspects of the present application are set out in the following numbered paragraphs (NPs)
NP 1. An implant removal device comprising:
   an elongate body having a proximal end and a distal end, the elongate body being resiliently flexible and configured to transmit torque from the proximal end to the distal end with a predetermined turning ratio; and
   a capturing structure extending distally from the distal end and having a capture region, the capturing structure being configured to selectively center a deployed implant in relation to a longitudinal axis of the elongate body and enclose the deployed implant.
NP 2. The implant removal device of NP 1, wherein the capturing structure comprises a braided conical structure having at least a portion of with a trumpet curved convex shape, a concave conical shape, or a straight conical shape.
NP 3. The implant removal device of NP 1, wherein the capturing structure comprises a finger conical structure.
NP 4. The implant removal device of NP 3, wherein an angular orientation of a distal end of a finger of the finger conical structure is between about 0 degree to about 45 degrees in relation to the longitudinal axis.
NP 5. The implant removal device of NP 3, wherein a portion of a finger of the finger conical structure has a radius of curvature of about 5 mm to about 50 mm in a deployed/pre-capture state.
NP 6. The implant removal device of NP 1, wherein the capturing structure comprises a double-wall conformable structure.
NP 7. The implant removal device of NP 1, wherein the capturing structure comprises a shape-memory material.
NP 8. The implant removal device of NP 1, wherein the distal end of the capturing structure having a cross-sectional major axis, in a direction transverse to a longitudinal axis of the elongate body, of about 1.5 times to about 15 times larger than a cross-sectional major axis of the proximal end.
NP 9. The implant removal device of NP 1, wherein the elongate body comprises a hypotube with a plurality cuts that control a flexibility of the elongate body.
NP 10. The implant removal device of NP 1, wherein the capturing structure is configured to apply a transverse force of about 0.5 Newtons to about 20 Newtons to the implant captured by the conical capturing structure, the force being transverse or radially compressive.
NP 11. An implant removal system comprising:
   an implant management tool configured for use in selectively separating an implant from tissue to which the implant is attached, the implant management tool comprising a distal opening with a cutting member configurated to selectively extend from the distal opening to cut the tissue; and
   an implant capturing device selectively extending from the distal end of the implant management tool, the implant capturing device comprising:
      an elongate body having a proximal end and a distal end, the elongate body being resiliently flexible; and
      a capturing structure extending distally from the distal end of the elongate body and having a capturing region, the capturing structure being configured to selectively center a deployed implant in relation to a longitudinal axis of the elongate body and enclose the deployed implant.
NP 12. The implant removal system of NP 11, wherein the implant management tool comprises a handle and a shaft extending distally from the handle.
NP 13. The implant removal system of NP 11, wherein the cutting member is disposed within the shaft.
NP 14. The implant removal system of NP 13, wherein the implant capturing device is slidably disposed within a lumen of the cutting member.
NP 15. The implant removal system of NP 14, wherein the cutting member is configured to slide along the implant capturing device and collapse the capturing structure.
NP 16. The implant removal device of NP 15, wherein the capturing structure comprises a braided conical structure having at least a portion with a trumpet curved convex shape, a concave conical shape, or a straight conical shape.
NP 17. The implant removal device of NP 15, wherein the capturing structure comprises a finger conical structure.
NP 18. The implant removal system of NP 15, wherein the cutting member comprises a cutting edge that extends at least partially around a circumferential edge of the distal end of the cutting member.
NP 19. The implant removal system of NP 11, wherein the implant removal device comprises an implant removal device from any one of NP 1-10.
NP 20. A method of removing an implant, the method comprising:
   advancing an implant removal device towards an implant deployed on tissue, the implant removable device comprising:
      an elongate body having a proximal end and a distal end, the elongate body being resiliently flexible and configured to transmit torque from the proximal end to the distal end with a predetermined turning ratio; and
      a capturing structure extending distally from the distal end and having a capture region, the capturing structure being configured to selectively center a deployed implant in relation to a longitudinal axis of the elongate body and enclose the deployed implant;
   capturing the implant with the capturing structure; and
   removing the implant from the patient.
NP 21. The method of NP 20, further comprising positioning an implant management tool toward the tissue.
NP 22. The method of NP 20, wherein capturing the implant further comprises torquing the elongate body to rotate the capturing structure and advance the capturing structure along a portion of tissue surrounding the implant.
NP 23. The method of NP 22, wherein the portion of the tissue surrounding the tissue is ingrowth tissue encapsulating the implant.
NP 24. The method of NP 20, wherein capturing the implant further comprises advancing a cutting member over the capturing structure to collapse the capturing structure.
NP 25. The method of NP 24, wherein the capturing structure comprises a braided structure.
NP 26. The method of NP 24, wherein the capturing structure comprises a plurality of fingers.
NP 27. The method of NP 20, further comprises separating the implant from the tissue.
NP 28. The method of NP 27, wherein the tissue is a valve leaflet.
NP 29. The method of NP 20, wherein the capturing structure comprise a braided capturing structure with a finger capturing structure disposed within the braided capturing structure.
NP 30. The method or NP 29, further comprising advancing a cutting member within the capturing structure to collapse the finger capturing structure.

## Claims

1. An implant removal device comprising:
an elongate body having a proximal end and a distal end, the elongate body being resiliently flexible; and
a capturing structure extending distally from the distal end of the elongate body and having a capture region, the capturing structure being configured to selectively center a deployed implant in relation to a longitudinal axis of the elongate body and enclose the deployed implant,
wherein the capturing structure comprises a conductive portion configured to deliver radiofrequency energy to tissue surrounding the deployed implant, and an insulative portion closer to the elongate body than the conductive portion.

2. The implant removal device of claim 1, wherein a braided member forms the conductive portion.

3. The implant removal device of claim 2, wherein the braided member extends along both the elongated body and the capturing structure.

4. The implant removal device of any preceding claim, wherein a proximal end of the implant removal device is electrically connected to a radiofrequency generator that provides the radiofrequency energy to the conductive portion.

5. The implant removal device of any preceding claim, wherein the capturing structure comprises a braided conical structure.

6. The implant removal device of claim 5, wherein at least a portion of the braided conical structure has a trumpet curved convex shape, a concave conical shape, or a straight conical shape.

7. The implant removal device of any preceding claim, wherein the capturing structure comprises a finger conical structure.

8. The implant removal device of claim 7, wherein the finger conical structure is disposed within the capturing structure.

9. The implant removal device of claim 7, wherein the elongate body includes a first elongate body extending from the braided capturing structure, and a second elongate body disposed within the first elongate body and extending from the finger conical structure.

10. The implant removal device of any preceding claim, wherein the capturing structure comprises a snare.

11. The implant removal device of any preceding claim, wherein the capturing structure comprises a shape-memory material.

12. The implant removal device of any preceding claim, wherein the elongate body is configured to transmit torque from the proximal end to the distal end with a predetermined turning ratio.

13. An implant removal system comprising:
an implant management tool configured for use in selectively separating an implant from tissue to which the implant is attached, the implant management tool comprising a distal opening; and
an implant removal device according to any of claims 1-12, the implant removal device configured to extend from the distal opening of the implant management tool.

14. The implant removal system of claim 13, wherein the implant management tool comprises a handle and a shaft extending distally from the handle.

15. The implant removal system of claim 14, wherein the shaft comprises a lumen, and the elongate body is configured to be extended and/or retracted along the lumen.
